# EUROPEAN PATENT APPLICATION

(11) **EP 4 299 001 A1**
(43) Date of publication of application: **03.01.2024**
(21) Application number: 22382601.7
(22) Date of filing: 27.06.2022
(51) Int. Cl.: A61B 5/0537

(54) **WEARABLE DEVICE, SYSTEM, AND METHOD FOR MONITORING HOMEOSTATIC STATE**

(71) Applicant: Universidad de Jaén, 23071 Jaén (ES)
(72) Inventor: Davydov, Dimitrii, Jaen (ES); Reyes Del Paso, Gustavo, Jaen (ES)
(74) Representative: Pons

(57) **Abstract**

The device (610) comprises: a bioimpedance unit (625) configured to periodically or irregularly sense a bioimpedance of at least one part of the body of the user via electrodes (635a-f) that touch a portion of the body around a limb or/and a portion of the body between the limbs of the user; a processing unit (605) that determines at least one physiological parameter of the user based on the bioimpedance and anthropometric data of the user and estimates, based on the at least one physiological parameter, a discrepancy between a current physiological state of the user and the homeostatic state of the user, its origin and compensation status; and an alert module (1050) that issues a message concerning the current physiological state of the user and an a recommendation for effective way of treatment of a homeostatic state decompensation.

## Description

### OBJECT OF THE INVENTION

The present disclosure relates generally to health monitoring and, more particularly, to devices, systems, and methods for monitoring a homeostatic state of a living organism.

### BACKGROUND OF THE INVENTION

Homeostasis is the state of a living organism at which the living organism maintains stable physical and chemical living conditions providing for optimal functioning of the organism. The conditions depend on many parameters, such as a blood pressure and blood volume, body temperature, concentration of sodium, potassium, and calcium ions in body tissues, concentration of glucose, oxygen, and carbon dioxide in blood, concentration of urea in blood, and so forth.

These parameters are controlled for being within their homeostatic ranges by respective regulatory systems of the living organism, such as food and drink behaviors (e.g., sodium and water intake), metabolic activity determining internal water production, perspiration (sweating) activity, renal function, mineralocorticoids, autonomic nervous system, and various humoral factors including angiotensin II, nitric oxide, and endothelin, using internal biosensors such as osmo-, baro-, volume-, chemo-, and thermo-receptors for detecting the parameters changes in responses to changes in environment, various physical and mental stress factors such as heating, cooling, injuries, threats, mental and physical activities such as cognitive and emotional tasks, exercises, body posture changes, painful stimuli, blood loss, and so forth.

The loss of capacity of the living organism to regulate properly (maintain or restore/recover) the parameters to their homeostatic states (i.e., a decompensated condition) may be indicative of a low resiliency against diseases, risk or predisposition to diseases or even an early stage of one or more diseases, as well as a risk of death. Accordingly, the parameters indicative of the homeostasis state, deviation from it, and functioning of its regulatory mechanisms are needed be monitored periodically or occasionally, in order to predict risks of one or more diseases or to provide immediate advice to restore the balance of parameters affecting the conditions of the living organism, especially in the cases of failure in the regulatory systems such as thirst sensation inadequacy in different populations in modern time.

For example, impaired homeostatic blood pressure control leads to essential or primary hypertension, a major risk factor for premature death and a leading risk factor for global disease burden. Basically, according to the "mosaic" hypothesis, primary hypertension is considered to be a single integrating or cumulative indicator of impaired whole-body health condition caused by the disruption of regulation of multiple other parameters within normal homeostatic ranges, including those maintaining body water/sodium balance, plasma glucose and urine levels, sympathetic/parasympathetic nervous system balance, and thermoregulation and affecting the main pressure supporting factors, such as cardiac output, peripheral vascular resistance, and blood volume.

This integrating homeostatic attribute of such parameter as blood pressure has been utilized by clinical doctors exploiting blood pressure monitors with various technologies, from manual (sphygmomanometers) to digital (with automatic inflation), in non-portable and portable form factors, with arm, wrist, or finger cuff-style and cuffless methods, with auscultatory (mercury and aneroid manometers) and oscillometric pressure reading methods for diagnosis of hypertension. Many efforts have been invested in the development of new more advanced technologies in this field.

However, the other side of this universal health-related parameter being detected by blood pressure monitors is that this measure cannot help to detect origin of blood pressure increase for its correct therapy, corresponding to a chronical bias of some related parameters such as increased or decreased blood volume size regulated by osmo- and volume (low pressure baro)-reflexes.

Indeed, blood pressure monitors show similar normal pressures in chronically hyperhydration state and in chronically hypohydration state if the states are compensated by such hemodynamic mechanisms, as a reset of high pressure baroreflex regulation to chronical vasodilation or chronical vasoconstriction, respectively. If hyperhydration or hypohydration state is decompensated, it leads to the similar blood pressure parameters clinically diagnosed in both cases as essential hypertension with unidentified pathophysiological hyperhydration or hypohydration origin. However, cases of essential hypertension with different decompensated increased or decreased blood or plasma volume as its causes are sensitive to different antihypertensive treatment using mainly either diuretics or vasodilators.

A resistant hypertension as a kind of hypertension with low efficiency of antihypertensive drugs (i.e., intake of at least three ones including a diuretic) in controlling blood pressure in up to 10% of patients treated for hypertension and associated with a high risk of cardiovascular morbidity and mortality can be an example of incorrect antihypertensive medication with reference only to blood pressure levels obtained by current blood pressure monitors without blood volume assessment. Burdening of healthcare service by hypertension treatment and a cost/effectiveness ratio of interventions using current antihypertensive treatment guidelines with 'trial-and-error' approach in antihypertensive drugs prescriptions and life-style recommendations such as salt-restricted diet are both very high.

This challenge claims development of a technology that assesses blood, plasma or extracellular water volume for predicting hyper- and hypo-hydration mechanisms of blood pressure increase, the risk for hyper- or hypo-volemic, 'salt-sensitive' or 'salt-resistant' essential hypertension, and medication outcomes in response to diuretics and vasodilators (i.e., responders and non-responders to specific antihypertensive therapy). The same technology should also help to adjust more correctly the dosage and course of antihypertensive treatment, because the initial response to the medications is counteracted, not only by the mechanisms balancing cardiac (via cardiac muscle contractility) and vascular (via vascular muscle contractility) control of blood pressure but, also includes independent volume control mechanisms as in response to diuretics in short- and long-term for partial or full buffering of their volume- and salt depletion effects using plasma renin and aldosterone activities sensitive to different antihypertensive medications and thus can be modified by effects not only from beta- and calcium blockers but also from blockers of the renin-angiotensin-aldosterone system (RAAS) (e.g., angiotensin-converting enzyme [ACE] inhibitors or angiotensin receptor blockers).

Monitors with Blood/Plasma Volume (BV) measurement technologies can be stand-alone devices (i.e., as BV monitors) or be implemented in the current or future blood pressure (BP) monitors and vice versa, as hybrid BV-BP monitors.

Studies conducted in healthy participants with between- and within-subject designs have shown that bioimpedance sensors with specific combinations of their segmental (including tissues over a central body part, the body trunk, or the body corpus) and local (i.e., restricted to peripheral tissues) body placements for the current passing through different specific tissues can be used to predict physiological origins of individual blood pressure level differences and blood pressure fluctuations, as responses to blood volume contraction, due to negative fluid balance with uncompensated (by cardiac output decrease) increase in peripheral vascular tone, or to a blood volume expansion with body water/sodium conservation due to positive fluid balance with uncompensated (by vasodilation) increase in cardiac output.

Previous studies showed that antihypertensive treatment according to a body water volume-control, assessed by current clinical bioimpedance devices, compared to the clinical blood pressure monitoring as recommended by the regular clinical guidelines could increase a decline in arterial stiffness, left ventricle hypertrophy, non-dipper hypertension, and systolic and diastolic blood pressure levels, an improvement in endothelial function, and a growth of survival rate at follow-up.

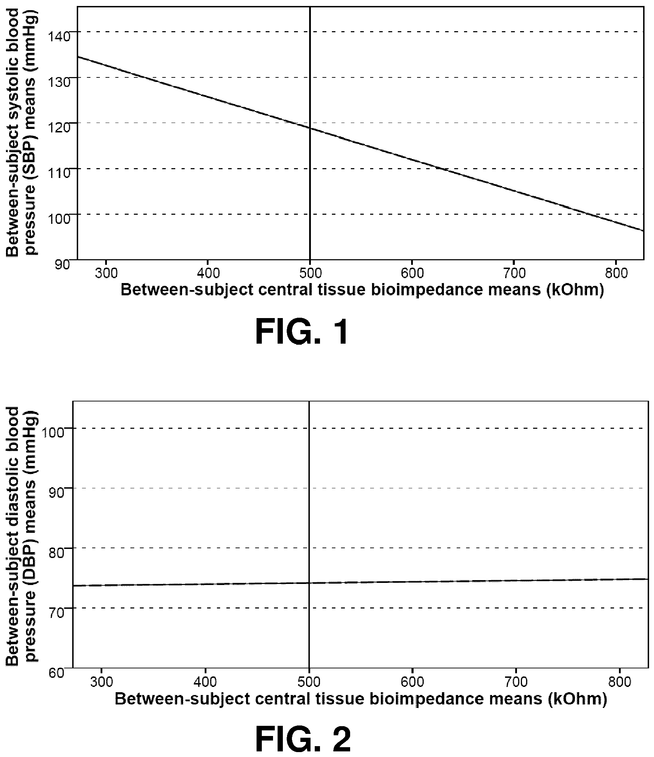

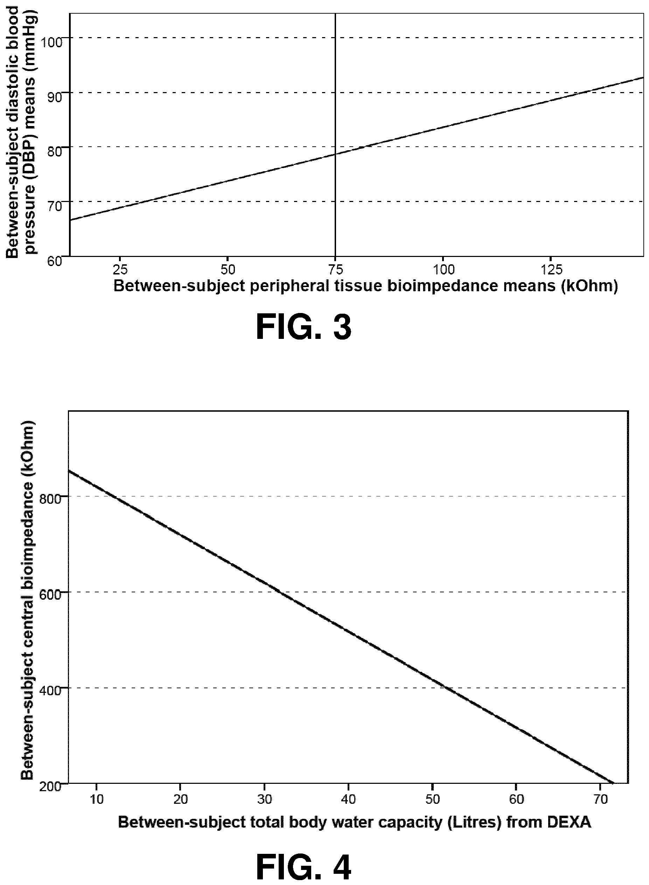

However, in these cases body water control was conducted (i) in laboratory or office settings and (ii) using extracellular (interstitial and intravascular) volume assessed by highly intercorrelated total body and thoracic bioimpedance measures of only central distribution of body water as the cause and severity of the hyper-hydration (hyper-volemic) type of essential hypertension (i.e., with blood volume expansion) without control for peripheral blood volume distribution as the cause and severity of the hypo-hydration (hypo-volemic) type of essential hypertension (i.e., with blood volume contraction) (Figures 1-3). Other devices and technologies for assessing body water capacity such as various isotope dilution techniques, dual-energy x-ray absorptiometry (DEXA or DXA), etc. look also not very reliable for distinguishing or separating central and peripheral blood or plasma volume effects on blood pressure regulation since are required adjustment for individual weight or height (Figures 4-7).

While impacts of the hyper- and hypo-volemic status as a component of circulation system on blood pressure level can be left unnoticed or unrecognized with only blood pressure measurement using current blood pressure monitors, correctly designed algorithms with correctly located bioimpedance sensors implemented in wearable mobile devices as stand-alone blood volume (BV) monitors or combined with blood pressure (BP) devices as hybrid BV-BP monitors can distinguish the body hydration conditions and be used for monitoring body hydration fluctuations and predicting its impairment as a main probable cause of hyper- or hypovolemic hypertension and other cardiovascular disorders (e.g., heart failure), metabolic and chronic pain syndromes, diabetes, pulmonary and renal diseases. These BV and BV-BP devices can be used for predicting medication effectiveness such as diuretics and vasodilators in patients with hypertension using the assessment of the volume-pressure relationships.

Central blood circulation or hemodynamics (i.e., activity of the central vascular system) associated with distribution of central blood, plasma or water volume can be assessed by a whole-body bioimpedance, segmental bioimpedance of thorax or trunk (e.g., derived from hand-to-hand, hand-to-arm, arm-to-arm, leg-to-arm, leg-to-hand, extremites-to-neck/head, or thoracic impedance measures). In this case, a lower whole body or segmental bioimpedance associated with an increased blood or water volume (i.e., hyperhydration) accounted to a higher venous blood return, a higher stroke volume, a higher heart rate, and a higher cardiac output predicts an increased systemic blood pressure due to these central hemodynamics effects (Figure 1) causing a Volhard's 'Red', or a Laragh's 'Wet' essential hypertension development as outcome if homeostasis is decompensated. In this case, diuretics are considered to be the most effective among antihypertensive medications as first-line treatment.

Peripheral blood circulation or hemodynamics (i.e., activity of the peripheral vascular system) associated with distribution of peripheral blood, plasma or water volume can be assessed by local bioimpedance of tissues in extremities (arms, legs) or other body parts with tissue well-vascularized by small and medium-size arteries and arterioles such as skeletal muscles. In this case, higher local tissue bioimpedance is associated with a decreased blood or water volume (i.e., hypohydration) effects accounted to a higher total peripheral vasoconstriction that predicts increased systemic blood pressure due to these peripheral hemodynamic effects (Figure 3) causing a Volhard's 'Pale', or a Laragh's 'Dry' essential hypertension development as outcome if homeostasis is decompensated. In this case, vasodilators are considered to be the most effective among antihypertensive medications as first-line treatment.

In general, the homeostatically compensated and decompensated distributions of water between central and peripheral tissues assessed by respective bioimpedance measures of central and local tissues can successfully be used for distinguishing health resilience and health risk conditions with respect to central hemodynamic salt-sensitive (hypervolemic, Volhard's 'Red', or Laragh's 'Wet') and with respect to peripheral hemodynamic salt-resistant (hypovolemic, Volhard's 'Pale', or Laragh's 'Dry') subtypes of blood pressure regulation and respective hypertensions which are sensitive to different medications (with natri/diuretic or vasodilation effects, respectively) and dietary recommendations (e.g., with or without salt restrictions, respectively) and to be prevalently distributed in different populations (either in those of black race, male sex, younger age, and with obese body composition or in those of white race, female sex, older age, and with lean body composition, respectively).

### DESCRIPTION OF THE INVENTION

The present invention describes a wearable device, system, and method for measuring, assessing, and monitoring a homeostatic state of a living organism.

Some embodiments of the present invention facilitate an early detection of risk for diseases or disorder development in a human organism. Some embodiments of the present disclosure facilitate an early detection of diseases or disorder in a human organism. Some embodiments of the present disclosure improve diagnosis of diseases or disorder in a human organism. Some embodiments of the present disclosure improve treatment and management of diseases or disorder in a human organism. Some embodiments of the present disclosure improve protection of a human organism (prophylaxis) against diseases or disorder development. Some embodiments of the present disclosure include providing a wearable device for monitoring bias from a homeostatic state of a user.

The wearable device may provide information about a level and regulation of hydration (water volume) of a human body locally (peripherally), centrally, and in general in real time, and assess the deviation from the homeostatic balance of water and electrolytes of the human body of a user of the wearable device, its origin and compensation status, and an at least one effective way of treatment of a homeostatic state decompensation, correction (recovery or restoration) of a homeostatic state deviation, protection of a homeostatic state from a deviation, and/or promotion of high homeostatic state resiliency (fast restoration and/or stability).

The level of hydration and the deviation from the homeostatic balance of water and electrolytes of the human body, with its origin and compensated and decompensated status of regulation can be determined based on the bioelectrical impedance (BIA) data collected with or without assistance of the user. The BIA is sensitive to the body fluid volume and its assessment of the peripheral and central volumes of water (i.e., absolute and relative water distribution, general and peripheral-to-central balance within the body, correspondence of changes in peripheral and central water volumes with high, low positive or negative cross-correlations, or their discordance in changes in long- and short-terms) are the main indicators, showing whether the homeostatic state of the human body is normal, as in case of blood pressure stability, its increase as a temporal and compensated deviation from the homeostatic state, or its increase as a permanent and decompensated deviation from the homeostatic state (i.e., a disease of the body such as hypertension).

The BIA can be used to monitor compensated and decompensated deviations from the homeostatic state of the body, which may be indicative of the state of health of the user, in general and with respect to cardiovascular, circulation, or hemodynamic system and hypertension as a negative outcome of decompensated blood/plasma volume changes with a water hyper- or hypo-volemic origin, and with recommendations of its correct management and treatment using either diuretics or vasodilators, salt-restricting or -unrestricting diets and so on.

The assessment of hydration of the body and its distribution as an individual homeostatic state with compensated and decompensated deviations from the homeostatic state of the body is different from the determination of water volume and water content in the body as one of body compartments along with other such as fat, bones, and proteins.

The wearable device of the present disclosure can assess the level of hydration and its compensated (a positive within-subject cross-correlation between volume measures of water distributed peripherally and centrally and between blood pressure and water volume measures in phase or phase-shifted) or decompensated (a negative within-subject cross-correlation between volume measures of water distributed peripherally and centrally or the absent of within-subject cross-correlation between blood pressure and water volume measures in phase or phase-shifted) deviations from the homeostatic state.

The level of hydration of the user is estimated based on individual data of the user. The individual data may include a level of dehydration and overhydration distributed in general, at peripheral tissues (i.e., related to peripheral vascular resistance and extracellular [intra-vascular and extra-vascular] water distribution), and centrally (venous blood return to the heart as its volume in the thorax) as a deviation of the individual state of hydration from the homeostatic state: water hypo-volemia peripherally (as a proxy of higher peripheral vascular resistance determining at first a higher diastolic blood pressure followed later by a higher systolic blood pressure) and water hyper-volemia centrally (as a proxy of a higher central blood return determining at first a higher cardiac output with high systolic blood pressure followed later by a higher diastolic blood pressure).

The individual data may further include a usual individual homeostatic level of hydration and amount of rehydrate or dehydrate needed to provide a return to the homeostatic level of hydration for a particular user, calculated with the reference to absolute water measures provided by such methods as DEXA (Dual-energy X-ray absorptiometry). These data are associated with individual homeostatic (i.e., healthy) mean and range of the hydration level calculated by such methods as the rhythm-adjusted mean or MESOR (a mean value based on the distribution of water volume values across the cycles of the user's activities, computed using a cosine function) with the double amplitude or 2A (a measure of the extent of predictable normal changes of water volume within the cycles of the user's activities) taken as reference values and its compensated or decompensated condition (indicated by coherent or concordant reactivity of blood pressure and water volume as well as peripheral and central water volume measures within the cycles of the user's activities) as opposed to simply calculating the amount of water in the body together with other body compartments, with reference to general or specific population data (i.e., the calculation performed in conventional tracking devices for monitoring the state of body compartments).

Additionally, in an assessment of hydration level, the following reference indicators can be used: a short-term or circadian change of a body weight, hematological parameters (e.g., a volume of plasma represented via the concentration of hemoglobin and hematocrit), levels of plasma osmolality/osmolarity and concentrations of sodium, urinary values (urine specific density and osmolality of urine), saliva values (osmolality/osmolarity and volume of saliva and saliva specific density), cardiovascular values (e.g., pattern with time and extent of clino- and orthostatic cardiovascular reactivities and recoveries including blood pressure, heart rate, stroke volume, cardiac output, pre-ejection time period, left ventricular ejection time, total peripheral or systemic vascular resistance), body temperature values, subjective values (e.g., dry mouth, thirst, and muscle weakness), sweating and peripheral blood flow activity related to vaso-constriction and - dilation (i.e., competing of homeostatic body thermoregulation with homeostatic body hydration mechanisms) in response to external challenges such as environmental temperature, altitude, bariatric, and gravity changes.

Conventional BIA of the total body water uses methods of radioisotope dilution with deuterium (or tritium) and sodium bromide or DEXA as references. In contrast to the conventional methods that estimate only an amount of water in the body, the methods for monitoring of deviation from the homeostatic state of the user of the present disclosure provide more personalized information, specifically levels of de-hydration (water loss with sweating, breathing, and diuresis) and re-hydration (water return with metabolism, drinking, and retention) during training sessions, hypo-hydration state as in some disorders such as hypo-volume essential hypertension and in aging progress, and overhydration state as in some disorders such as hyper-volume essential hypertension, edema and pre-eclampsia in pregnancy and edema in heart and renal diseases (e.g., heart failure).

Sports organizations may monitor levels of hydration and its compensated and decompensated deviation from a homeostatic state (dehydration, eu-hydration, and rehydration) with identified origin and recommendations for coping and protecting during training of athletes to avoid past situations in which players would get sick acutely in hot weather, a chronic disorder such as hypertension and heart failure, or even die.

Additionally, the levels of hydration and its deviations with identified origin and recommendations for coping and protecting can be useful during home monitoring of high-risk patients with decompensated hypo- or over-hydration states, as in patients with water hypovolemic or hypervolemic hypertension, respectively. Furthermore, besides hypertension, the overhydration state also needs to be known in the following pathological states: pregnancy (preeclampsia), respiratory, other cardiovascular (e.g., heart failure) and renal diseases, opioid overdose, and so forth.

The health-oriented approach of the present disclosure differs from fitness tracking and conventional medical approaches in wearable devices. Firstly, a more personalized approach in the assessment of compensated and decompensated deviation of individual health regulation mechanisms with identified origin and recommendations for coping and protecting is provided as compared to existing fitness tracking technologies not taking into account that a higher physical fitness may be harmful to health for the particular person.

Secondly, no medical examinations and certifications are required to indicate an individual homeostatic state, origins of its deviation and compensation status condition (e.g., compensated and decompensated volumetric mechanisms of blood pressure regulation), and with recommendation of an at least one effective way of treatment of a homeostatic state decompensation, correction (recovery or restoration) of a homeostatic state deviation, protection of a homeostatic state, and/or promotion of homeostatic state resiliency, as compared to conventional medical approaches with only the 'trial and error' approach in a disease treatment, such as in case of hypertension, without paying attention to individual homeostatic states and with the application only in patients but not in general populations and burdening healthcare system by its high cost and low effectiveness.

Approaches of the present disclosure also relate to predictive technologies, also called preventive technologies, as it indicates individual risks for hypertension in mechanisms decompensating a homeostatic state following compensated hypo- or hyper-volemic normotension states. Using these approaches, the operation of the wearable device can be based on the BIA data of the human body. An example method can be implemented in a wireless self-contained device, which may be attached to a limb, limbs, or other body parts of a user with regions with well-vascularized tissues such as skeletal muscles and provide comfortable long-term wearing.

Existing compact wearable hydration measurement devices may have 2 or 4 electrodes. However, the locations of the electrodes are a disadvantage of the existing devices. Specifically, the electrodes are typically located as close to each other as possible, thus limiting the field of application of the electrodes to measuring only skin conductance or impedance. This only allows measuring the level of skin hydration level determined by skin moistening or water accumulation in epidermis (e.g., the stratum corneum) via, e.g., sweating mechanisms, which is only weakly and indirectly related to peripheral vascular activity and a body hydration state as a homeostatic condition determining hypo-volemic mechanism of increased blood pressure.

In the existing devices, a moisture meter having a penetration capacity of up to 5 mm is usually used as a reference device for measuring skin hydration or water accumulation restricted to epidermis (e.g., the stratum corneum). Thus, the existing devices are incapable of measuring the hydration or water volume associated with the central cardiac and/or peripheral vascular activities and thus with blood pressure regulation, for example, in arteries and arterioles of muscles of users due to the location of the muscle tissues deeper under the skin and in large vessels of the thorax as located beyond the place of the current transmitted between the electrodes.

Other existing compact wearable hydration measurement devices may also have 2 or 4 electrodes. However, the locations of the electrodes (e.g., for a segmental hand-to-hand BIA or a local BIA measure of a portion of the human body) are used specifically for a water volume assessment as a compartment of body along with fat, muscles, and bones with reference to the total body compartments as assessed by DEXA, or as a water deficiency with assessment of a total water volume in tissues corrected to blood volume as a compartment of extracellular fluid volume with reference to other variables such as hematocrit.

Thus, these technologies do not distinguish peripheral and central water volume distributions, origin of their relative and absolute deficit, and its compensated and decompensated status with phase-shifted and unshifted effect on blood pressure level regulation, thus limiting the field of application of the devices to measuring mainly a total water capacity of body of the user. The electrodes are typically located at wrist in a watch-like device or contact a palm or fingers.

Without reference to a peripheral water volume and its daily, weekly, and monthly negative or positive cross-correlations with the central water volume changes the existing technologies cannot assess the compensation status of an individual homeostatic state of water balance and its effect on blood pressure regulation determining a risk for hypo- or hyper-volemic mechanisms of hypertension development and predicting an effective treatment approach with such medications as diuretics, beta-adrenergic blocking agents, calcium channel blockers, vasodilators, ACE inhibitors, and so on.

Thus, these existing devices are also incapable of measuring the hydration or water volume associated with the peripheral vascular activity determining hypo-volemic blood pressure regulation, for example, in muscles of users due to the transmission of the current in long circuit through the thorax with its stronger water volume effect on BIA measures hiding the modulating water effect of local tissues on the BIA measures associated with peripheral vascular activity.

Other existing devices may also have 2 or 4 electrodes to calculate coherence between physiological and behavioral variables. However, in the present disclosure coherence, phase, cross-correlation, or similar analysis is used to detect correspondence of at least two physiological or physiological and behavioral/environmental processes to predict a compensated or not compensated homeostatic state as a cause of health problems such as essential hypertension compared to existing technologies with detection of the correspondence of the reactivity measures or variables as an indicator of body's capability to effectively cope with challenges as an indicator of its health resilience to withstand to risks of future health problems.

No knowledge is available of whether compensation status of a homeostatic state deviation as detected by the presented correspondence metrics is related to the effectivity in coping with challenges as measured by correspondence metrics in the existing technologies and, if they are related, to what extent and how directly and whether recommendations for homeostatic state correction and recovery of the present disclosure could be relevant for improving the effectiveness of coping with challenges as measured by the existing technologies.

Thus, the present disclosure of the wearable device is not to combine any previous technologies such as skin hydration or moisture assessment, segmental (arm-to-arm or hand-to-hand) method of body composition assessment, and simply a local tissue hydration state evaluation with the application of specific coherent or cross-correlation analysis. Using an example of the assessment of volumetric mechanisms of blood pressure regulation and hypertension development the present invention is to show a way of application (with selection of measurement techniques with correct designs, protocols, procedures, and mathematical analysis) of scientifically uncovered (by hypothesis- and/or data-driven research) evolutionary established physiological mechanisms of a homeostatic state regulation (such as central and peripheral water balance regulation for maintaining blood pressure) on some conventional technologies such as BIA for their translation to more advanced technologies for individual homeostatic states evaluation and tracking to assess or predict mechanisms and risks of compensated and decompensated deviations from the homeostatic states and proper methods of their corrections and treatments (such as in essential hypertension with different volumetric mechanisms of its development).

The wearable device of the present disclosure provides a measurement of the peripheral and central hydration levels, its relative and absolute balance in distribution, with the balance coordination as positively or negatively coupled (coherent) or uncoupled (not coherent). This physiologically coordinated or not coordinated distribution of blood, plasma or water volumes between the body parts may be the assessment of compensated and decompensated homeostatic states associated with regulation of blood pressure of the body, using a property of body tissues to resist to high frequency current (bioimpedance) depending on the volume of mainly extracellular or intravascular water with electrolytes in blood plasma, also regulated by the concentration of dissolved particles per a unit volume (osmolality).

The extracellular or intravascular water volume and its osmolality are regulated by separate mechanisms associated with high pressure baro-receptors, low pressure baro (or volume)-receptors, and osmoreceptors and may have different impact on mechanisms of homeostatic state compensation with further impact on different volumetric status of essential hypertension if it is decompensated.

The advantage of the wearable device of the present disclosure is the ability to track deviations from the homeostatic state of the user body which may initiate the development of the disorder if they are not compensated by respective counteracting physiological resilience mechanisms. The information provided by the wearable device of the present disclosure is more detailed in mechanisms regulating homeostasis and useful in other areas of healthcare, as compared to the data provided by conventional clinical or existing wearable devices, as no one of known technologies and devices can assess volumetric origin of essential hypertension and predict efficiency of its treatment that is critically essential to human health in general as it is proposed by an example embodiment.

In a first aspect of the invention, the wearable device for monitoring a homeostatic state of a user is described. The device comprises bioimpedance sensors configured to sense periodically or in response of irregular automatic or manual trigger a bioimpedance, of at least one tissue of a user, via a first set of electrodes intended to touch a portion of the body around a limb locally or other parts of the body with regions with well-vascularized tissues such as skeletal muscles of the user, and a bioimpedance of whole-body or segmental (thorax or trunk) of the user via a second set of electrodes, intended to touch tissues around the respective central parts of the body of the user (e.g., as in between-arms bioimpedance, in impedance cardiography, and in whole body bioimpedance configurations).

Sensing the bioimpedance periodically or non-periodically depends on the application for which the device is being used. On the one hand, regular bioimpedance measuring is associated, with its more or less frequent timing, with some related circadian processes such as diurnal homeostatic body water regulation associated with MESOR (Midline Statistic of Rhythm) values. Frequency of its timing is related to local climate, season, and individual health, occupation, physical, social, food/diet behaviors and so on. On the other hand, irregular measuring is associated with manual measurement on demands (e.g., related to medication intake, food/fluid intake) and/or with automatic measurement related to some predefined events such as position changes (e.g., as in clino-orthostatic challenge) detected by accelerometers and/or gyroscope, weather changes detected by barometer or taken from respective online services, physiological or pathological events such as heart rate changes detected by electro-, photo- and/or other sensors, variations in physical activity detected by accelerometers, gyroscope, GPS, and/or alike sensors and so on.

The wearable device further comprises a processing unit communicatively coupled to the bioimpedance sensors. The processing unit comprises a memory module, that stores anthropometric data of the user, that may include one or more of the following: user's body weight, height, age, and sex, as well as a distance between the electrodes, circumference of the portion of the body part, sectional area, and so forth.

The processing unit also comprises a physiological parameter calculation module, that calculates at least one physiological parameter of the user based on the bioimpedance (obtained with the sets of electrodes) or on the bioimpedance and anthropometric data of the user stored in the memory module.

The physiological parameters may include an absolute or relative volume of fluids (water) in tissues within a portion of the body part of the user such as a limb and the thorax. In some embodiments, the at least one physiological parameter may include a level of hydration with balance of water and electrolytes in tissues within a portion of the body part of the user such as a limb and the thorax. In some embodiments, the at least one physiological parameter may include a level of compensated or decompensated correspondence of water or hydration balance between the tissues within a portion of the body part of the user such as a limb and the tissues and organs within the thorax or trunk measured by cross-correlation or coherence coefficients.

The at least one physiological parameter may be determined using a mathematical (e.g., regression) model obtained from a range of between-subjects measures in population (a population reference method) or from a range of within-subject measures in the individual (individual reference method). The model may be generated based on data sets of values of the bioimpedance, values of the anthropological data, and values of the at least one physiological parameter.

The processing unit may further comprise a discrepancy calculation module that estimates, based on the at least one physiological parameter, a discrepancy between a current physiological state of the user and the usual homeostatic state of the user, its origin and compensation status, and an at least one effective way of treatment of a homeostatic state decompensation, correction (recovery or restoration) of a homeostatic state deviation, protection of a normal homeostatic state, and/or promotion of homeostatic state resiliency against future challenges.

Additionally, the processing unit comprises an alert module, connected to the discrepancy calculation module that, based on the discrepancy existence and extent information, it issues at least one alert or message with recommendations concerning the current physiological state of the user. Specifically, the discrepancy calculation module may be configured to determine, based on the absolute or relative levels of total, regional (segmental), and local hydration with either compensated (i.e., without blood pressure increase) or decompensated (i.e., with blood pressure increase) regulation, and an amount of body water to dehydrate or to rehydrate for removing the discrepancy in the homeostatic state.

In this embodiment, the at least one alert or message may include at least a recommendation to the user to take the actions to rehydrate (e.g., to take a respective amount of the solute with particular electrolyte content or of pure water) or to take the substances (e.g., specific diuretics) or actions (e.g. thermal bathing or exercises) to loss the respective amount of solute or water volume (i.e., to dehydrate). If the user is a patient with hypertension the at least one alert or message may include at least a recommendation to the user to take a vasodilator or a diuretic to transfer the decompensated state of water disbalance to compensated or to bring hydration status to water balance according to individual water expansion or water constriction status.

In an embodiment, the wearable device may further include a support layer or layers holding the bioimpedance sensors and the processing unit. The support layer may be configured to be secured around the limb or another body part of the user with regions with well-vascularized tissues such as skeletal muscles.

The wearable device may further comprise a communication unit. The communication unit is configured to transmit data, including the at least one physiological parameter, to an external computing system.

In a second aspect of the invention, a method for monitoring bias from a homeostatic state of a user is provided. The method comprises a first step of periodically or irregularly sensing a bioimpedance of the user. The bioimpedance may be sensed by bioimpedance sensors and via internal electrodes configured to touch various sides of a limb of the user for local tissue bioimpedance measurements to calculate a peripheral circulation system status and via internal and external electrodes configured for their touching of a side of one limb of the user and their touching by another limb of the user for between-arms (i.e., segmental) bioimpedance measurements as a proxy of thorax bioimpedance measurements to calculate a central circulation system status.

A second step of the method is determining at least one physiological parameter of the user by a processing unit communicatively coupled to the bioimpedance sensor. The at least one physiological parameter of the user may be determined based on the bioimpedance or on the bioimpedance and anthropometric data of the user.

The values of the at least one physiological parameter may be obtained using a reference method. The reference methods may be divided to two main categories: reference ranges of absolute values of a homeostatic variable and reference ranges of relative values of bias from a homeostatic midpoint. Because a homeostatic midpoint variable can vary between subjects its absolute value fluctuation may not be very informative with the reference to population range in the assessment and prediction of health and disease of a particular individual because of a well-known problem in "precision" and "personalized" medicine approaches to obtained a specific granular normal reference population for every individual from demographically diverse population strata (including race/ancestry, gender/sex, age, and socioeconomic strata of the population).

A relative bias from an individual homeostatic midpoint assessed by specific mathematical methods such as cross-correlation, coherence or phase analysis between physiological parameters accumulated in response to respective (e.g., de-, eu- and re-hydration) conditions or in response to other behavioral activities (e.g., clino-orthostatic, circadian or menstrual cycle challenges) can be more robust in the assessment and prediction of health and disease of a particular individual.

The first category of reference methods can include computer scanning such as dual-energy x-ray absorptiometry (DEXA) and isotope dilution techniques. Another category of reference methods includes such measures that explore within-subject individual homeostasis-related compensated or decompensated effects of body water status changes between hyper-, eu- and hypo-hydration conditions induced by natural (e.g., biological cycles such as circadian, menstrual and so on) or artificial (such as physical exercises, thermal and cooling conditions, substance intake with water loss as diuretics, water retention as salts, or metabolic water regulation as capsaicin effects, gravity stress and so on) causes on physiological functions related to delay, onset threshold, and amplitude/magnitude in changes of mean of blood pressure, heart rate, sweating, diuresis, skin vasodilation, and so on, as well as of their responses (reactivity) to physical (e.g., orthostatic and handgrip) challenges, deep breath-in and breath holding probe, immersion in water probe, drinking probe, long or short local, segmental, or whole-body heating probe, short local alternating or direct current application probe and so on. The latter category of reference methods can only be developed using technologies implemented in wearable devices for ambulatory or ecologically-obtained series of variables.

A third step of the method is estimating, by the processing unit, a discrepancy between a current physiological state of the user and the usual homeostatic state of the user in general, for the same condition (e.g., resting, exercising, working and so on), or same daytime (morning, afternoon, night), time period, week day, month, season and so on, same place or surroundings (e.g., geographical, social, in/out a house and so on), or for a general or specific population reference. The estimation may be performed based on the at least one physiological parameter with further discovery of its origin and compensation status condition.

A fourth step of the method is issuing, by the processing unit and based on the discrepancy, at least one alert or message with recommendations concerning the current physiological state of the user.

Local (regional or peripheral) and central (e.g., segmental arm-to-arm or whole-body) bioimpedance measurements to obtain the at least one physiological parameter can be conducted both in the same measurement trials or sessions or each separately in their own measurement trials or sessions.

### DESCRIPTION OF THE DRAWINGS

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:
Figures 1-3.- Show predicted relationships of between-subject central and peripheral bioimpedance measures with between-subject homeostatic states of systolic (as a measure mainly centrally regulated by blood/water return, stroke volume, heart rate, and cardiac output variables; Figure 1) and diastolic (as a measure mainly regulated peripherally by the total peripheral vascular resistance; Figure 2 and Figure 3) blood pressure measures, calculated from experimental data obtained according to some example embodiments.
Figures 4-5.- Show predicted relationships of a between-subject homeostatic state of total water capacity/volume (in liters) obtained using a dual-energy x-ray absorptiometry (DEXA) with between-subject central and peripheral bioimpedance measures, calculated from other experimental data obtained according to some example embodiments.

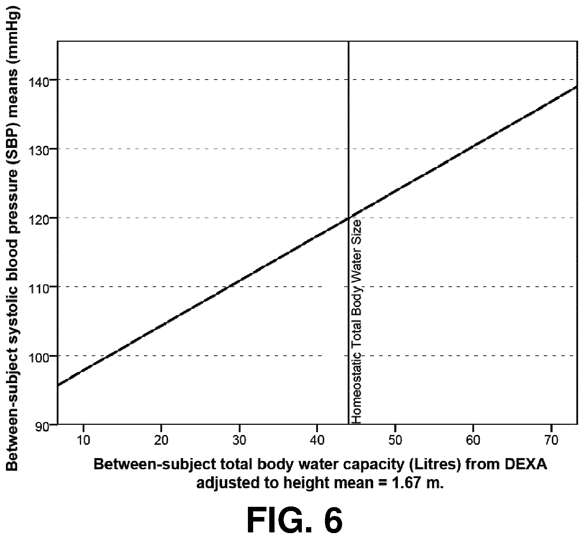

Figures 6-7.- Show predicted relationships of between-subject homeostatic states of total water capacity/volume (in liters) obtained using the dual-energy x-ray absorptiometry (DEXA) with between-subject homeostatic states of systolic and diastolic blood pressure measures adjusted to height or weight, respectively, calculated from other experimental data obtained according to some example embodiments.

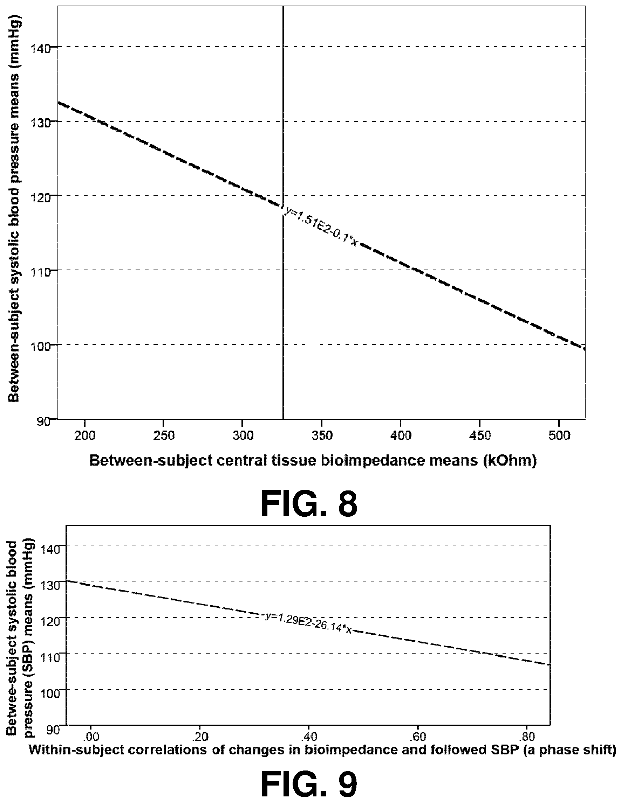

Figure 8.- Shows predicted relationships of a between-subject central bioimpedance measure with between-subject homeostatic states of systolic blood pressure measure, calculated from other experimental data obtained according to some example embodiments.
Figures 9-12.- Shows predicted relationships of a within-subject correspondence (correlations from -1 to +1) of central and peripheral bioimpedance changes in response to homeostatic body hydration state challenges (de- and re-hydration manipulations) or within-subject correspondence (correlations from -1 to +1) of bioimpedance and systolic blood pressure changes in response to the same hydration state challenges with between-subject homeostatic states of systolic and diastolic blood pressures, and heart rate (regulated centrally by autonomic nervous system) measures, calculated from other experimental data obtained according to some example embodiments.

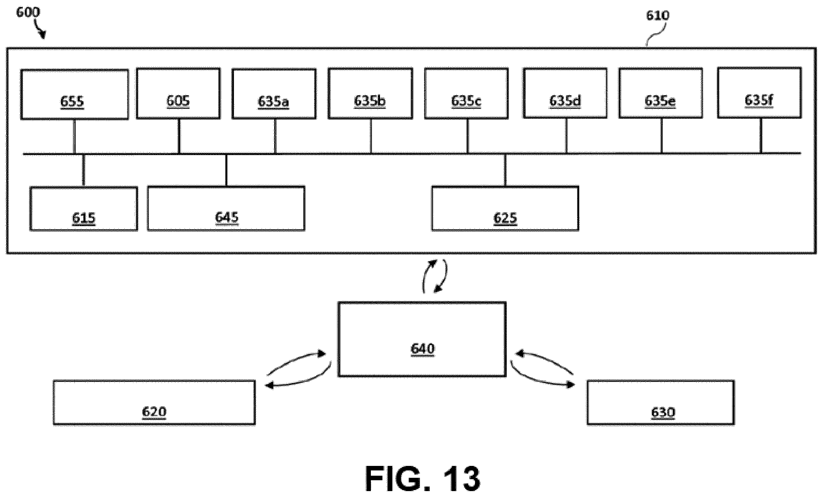

Figure 13.- Shows a block diagram of an environment, wherein methods for monitoring bias from a homeostatic state can be implemented, according to some example embodiments.

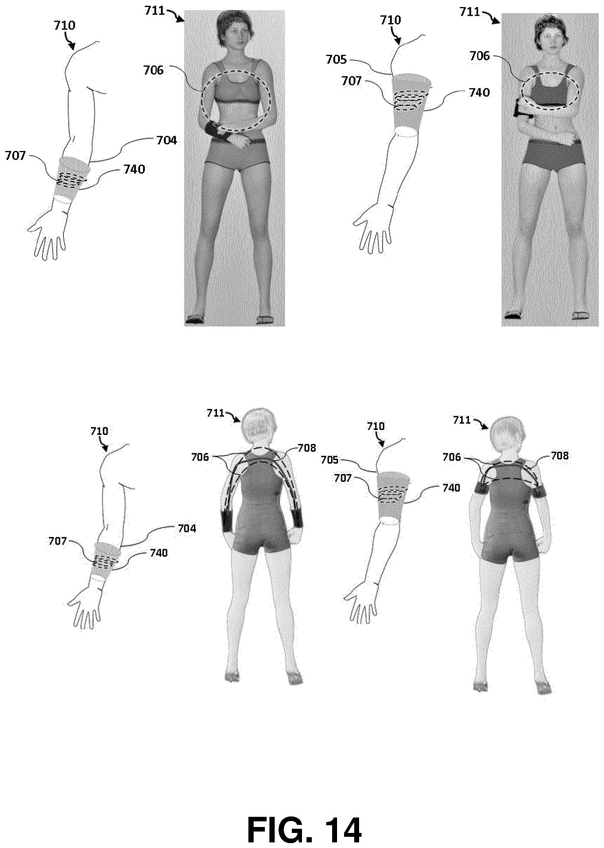

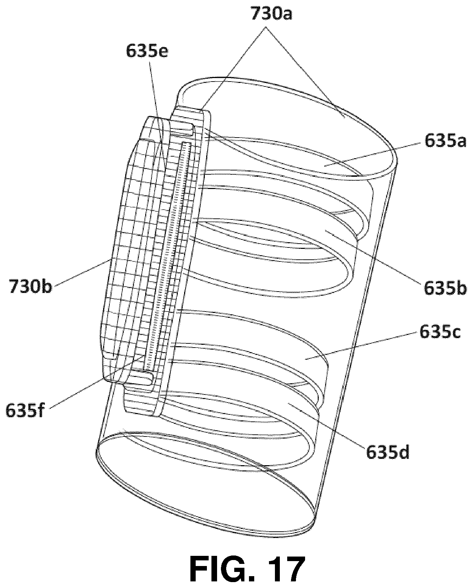

Figure 14-17.- Show locations of electrodes for measuring a local bioimpedance of tissues of a body in a short circuit (e.g., a limb in its upper or lower region [the shoulder or forearm] with well-vascularized skeletal muscles) and in a long circuit through the thorax (e.g., from arm-to-hand or arm-to-arm in their upper and/or lower regions) equipped in an armband, wrist-guard, or bracer.

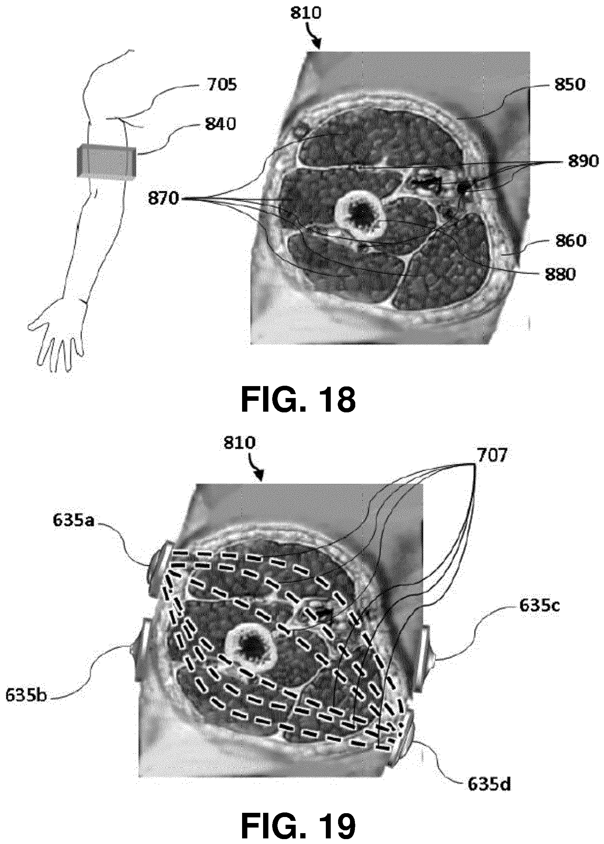

Figure 18.- Shows a section of the shoulder with well-vascularized skeletal muscles.
Figure 19.- Shows the same section of the shoulder as in Figure 18 with locations of internal metal plate electrodes) for measuring a local, regional, or peripheral bioimpedance of tissues of a body in a short circuit, according to an example embodiment.

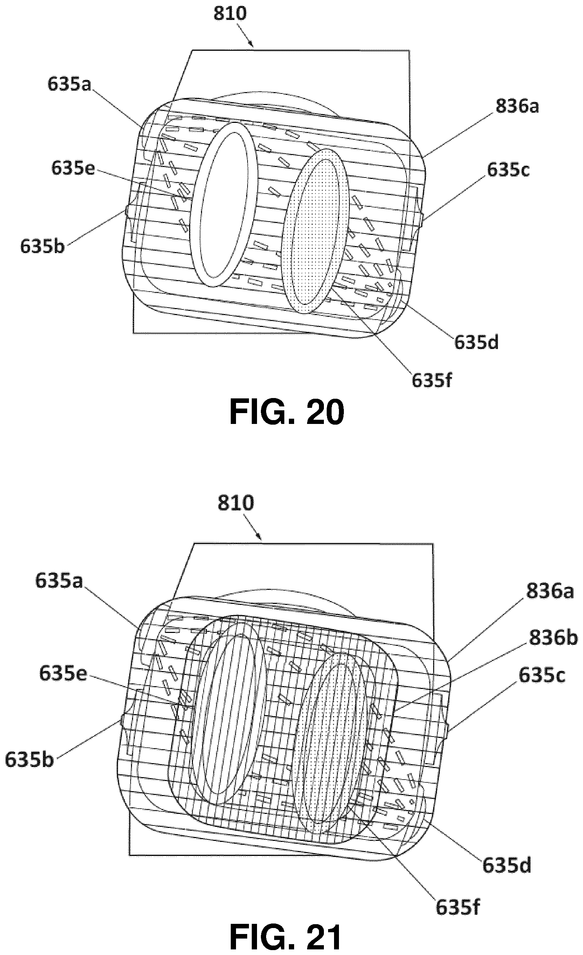

Figure 20.- Shows the same location as in Figures 18 and 19 with an elastic covering material from non-conductive plastic, fabric, or textile used to isolate and protect (secure) the internal electrodes and the body skin under them from the contact with external electrodes, and other body parts contacted the external electrodes for measuring a central bioimpedance of tissues of a body in a long circuit.
Figure 21.- Shows the same location as in Figures 18-20 with an elastic covering material from non-conductive plastic, fabric, or textile made in the form of a pocket, glove, mitt, or a 'female' sleeve, for placing the second hand there and holding/fixating it to keep in a stationary state when contacting with the external/outer electrodes during central bioimpedance measurement of tissues of a body in a long circuit.

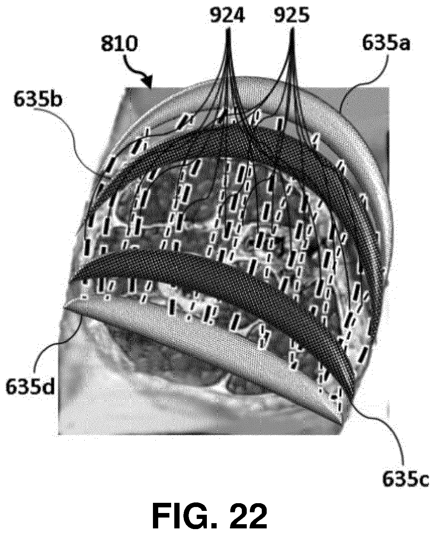

Figure 22.- Shows the same location as in Figures 18-21 but with flexible internal electrodes manufactured with an elastic textile pad and with an integrated logic circuit that allow an adjustable and flexible placement covering a limb around as a band or a strap, for measuring local bioimpedance of tissues of a body in a short circuit with various superficial and deeper current, according to an example embodiment.

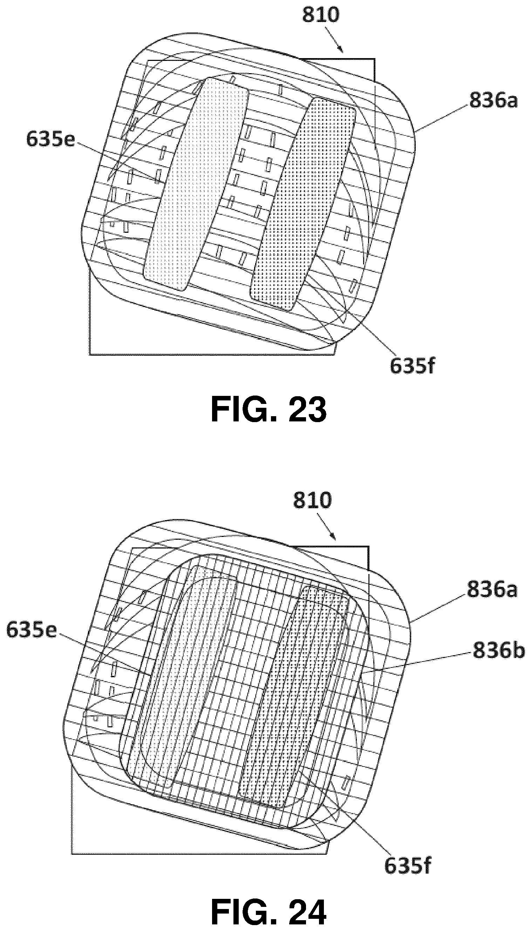

Figure 23.- Shows the same location as in Figure 22 with an elastic covering material from non-conductive plastic, fabric, or textile used to isolate and protect (secure) the internal electrodes and the body skin under them from the contact with external electrodes, and other body parts contacted the external electrodes for measuring a central bioimpedance of tissues of a body in a long circuit.
Figure 24.- Shows the same location as in Figures 22-23 with an elastic covering material from non-conductive plastic, fabric, or textile, made in the form of a pocket, glove, mitt, or 'female' sleeve, for placing the second hand there and holding/fixating it to keep in a stationary state when contacting with the external/outer electrodes during central bioimpedance measurement of tissues of a body in a long circuit.

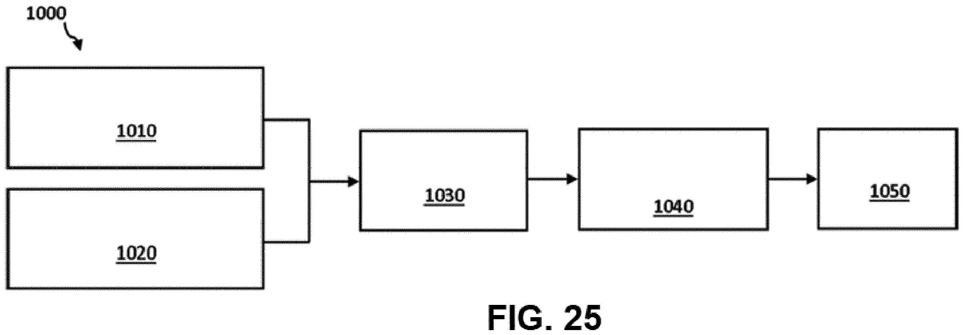

Figure 25.- Shows a system for monitoring a homeostatic state, according to an example embodiment.

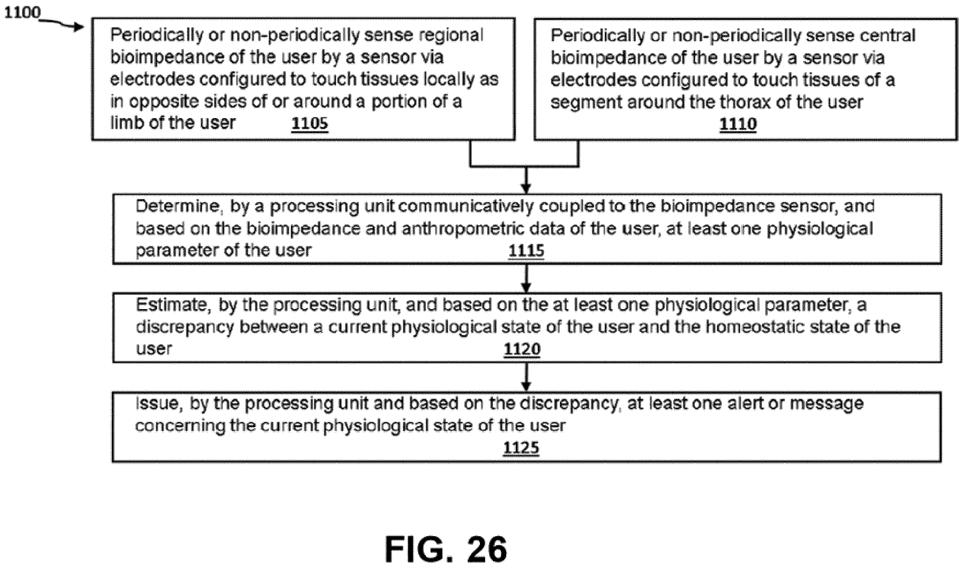

Figure 26.- Shows a flow chart illustrating an example method for monitoring a homeostatic state, according to some example embodiments.

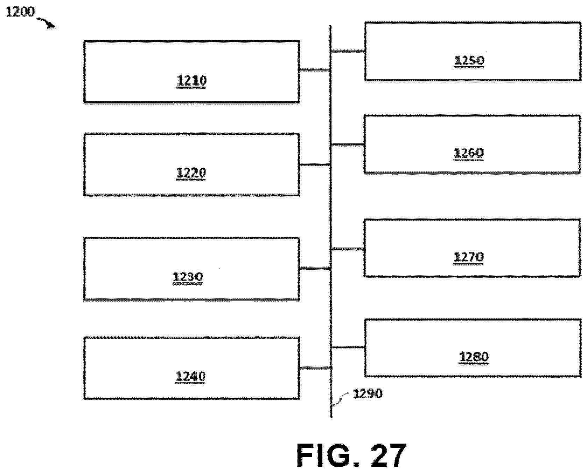

Figure 27.- Shows a computing system that can be used to implement a method for monitoring bias from a homeostatic state, according to an example embodiment.

### PREFERRED EMBODIMENT OF THE INVENTION

It is described below, with the aid of figures 1-27, a preferred embodiment of the wearable device, system, and method for monitoring homeostatic state, objects of the present invention. According to an example embodiment, the wearable device (610) for monitoring of a homeostatic state of a user comprises a bioimpedance unit (625), a processing unit (605) communicatively coupled to the bioimpedance unit (625), and a communication unit (645) communicatively coupled to the processing (605) unit as shown in Figure 13.

The bioimpedance unit (625) may regularly or irregularly sense bioimpedance of the user via electrodes (635a, 635b, 635c, 365e, 635f) configured to touch opposite sides of a limb of the user or to touch a limb around it. Based on the bioimpedance or on the bioimpedance and anthropometric data of the user, the processing unit (605) may determine at least one physiological parameter of the user. Based on the at least one physiological parameter, the processing unit (605) may estimate a discrepancy between the current physiological state of the user and the usual homeostatic state of the user assessed with the reference to a populational data or to individual MESOR and 2A data. Based on the at least two physiological or at least one physiological and at least one behavioral or environmental parameters, the processing unit may estimate their correspondence or discordance in activity (e.g., assessed by coherence, cross-correlation, and phase measures) as an indicator of compensated or not compensated discrepancy between the current physiological state of the user and the usual homeostatic state of the user. Based on the discrepancy and its details (the origin as hypo- or hyper-volemic and a (de)compensation condition), the processing unit (605) may issue at least one alert or message concerning the current physiological state of the user and recommendations of its treatment, correction, or protection. The communication unit (645) may transmit the at least one physiological parameter to an external computing device (630).

Figure 13 is a block diagram of an environment (600), wherein methods for monitoring deviation from the homeostatic state of a user, delivering information of its origin, compensation status condition, and an at least one effective way of treatment of a homeostatic state decompensation, correction (recovery or restoration) of a homeostatic state deviation, protection of a homeostatic state, and/or promotion of homeostatic state resiliency against current and/or future challenges or events can be implemented, according to some example embodiments.

The environment (600) may include a wearable device (610) for monitoring deviation from the homeostatic state of a user, a server or computing cloud (620), a computing device (630), and a network (640) for obtaining and delivering information of its origin, compensation status condition, and an at least one effective way of treatment of a homeostatic state decompensation, correction (recovery or restoration) of a homeostatic state deviation, protection of a homeostatic state, and/or promotion of homeostatic state resiliency against current and/or future challenges or events.

The wearable device (610) may comprise a bioimpedance unit (625) with several electrodes (635a, 635b, 635c, 635d, 635e, 635f), a processing unit (605) which in turn comprises a memory module (615), a communication unit (645), a power supply (655). The wearable device (610) may further comprise wires connected to the electrodes (635a-635f). The electrodes (635a-635f) can be configured to sense signals indicative of vital signs and other medical parameters of the human body. The bioimpedance unit (625) may be configured to regularly and/or irregularly sense a bioimpedance of the user via the electrodes (635a-635f).

A first set of electrodes (635a, 635b, 635c, 635d) may touch opposite sides of a limb of the user or to touch a limb around it to make a short (local) circuit (707) or (924/925) for the impedance measurement; a second set of electrodes (635e, 635f) may be touched by or connected to another limb of the body of the user to make a long (segmental hand-to-hand, hand-to-arm, or arm-to-arm) circuit (706) for the impedance measurement (Figures 14, 18 and 22).

Particularly, regarding the number of electrodes (635a-635f), the simplest embodiment for assessing both types of hypertension would have six electrodes (635a-635f), since it should be two circuits with four-pair electrodes for separately central and local (peripheral) body water assessment (i.e., hyper-hydration and hypo-hydration types of hypertension, respectively), where one pair can be used for both circuits.

If one wants only to assess either body central, or local water state (i.e., either hyper-hydration, or hypo-hydration type of hypertension, respectively) the simplest embodiment can have four electrodes (635a-635f).

For detecting and assessing a hypo-volemic type of hypertension, two pairs of electrodes (635a-635f) (including those measuring voltage) should allow current to pass through muscles supplied with small muscular arteries and arterioles that regulate the so-called "Total Peripheral Resistance" or "Systemic Vascular Resistance". Suggested positions are upper (one of arms) or lower (one of legs) extremities or limbs.

In this case, current goes from a pair of electrodes (635a-635f) (including one measuring voltage) placed on one side of the same limb through muscles to another pair of electrodes (635a-635f) (including one measuring voltage) placed on another side of the same limb. In an arm it can be the forearm (i.e., the lower half of the arm) or the brachium (i.e., the upper half of the arm).

For detecting and assessing a hyper-volemic type of hypertension, positions of two pairs of electrodes (635a-635f) (including one measuring voltage) should allow current to pass through the thorax with cardiac chambers, large veins, aorta and big elastic arteries that are related to blood return, stroke volume, and cardiac output.

Some suggested positions are two upper extremities or limbs (two arms or an arm and a hand of different upper extremities). In this case current goes from a pair of electrodes (including one measuring voltage) placed on one arm through the thorax to another pair of electrodes (including one measuring voltage) placed on another arm or contacted by the hand of another extremity. In the arms it can be the forearms (i.e., the lower halves of the arms) or the brachiums (i.e., the upper halves of the arm). The positions can be mixed, i.e., from the forearm of one arm to the brachium of another arm or from the hand of one upper extremity to forearm or brachium of another upper extremity.

The bioimpedance unit (625) may also be configured to detect a short-term change (e.g., in milliseconds, seconds, minutes, hours) in electrical impedance of the human body caused by the pressure pulse, volume pulse, blood flow, or arterial wall distention changes when the heart pumps blood to the periphery with modulation effects of the lungs activity (respiratory movements as a pump for venous return and respiratory sinus arrhythmia for more effective blood oxygenation) and various other body (e.g., metabolic- and gravity-related reflective) changes on long-term change in volume and osmolality of water in body tissues of the user. This detection may improve the prediction of long-term effects of hydration status on homeostatic state and to assess the impact of other compensation mechanisms on its resilience or resiliency to deviations.

The bioimpedance unit (625) may provide a bioimpedance signal. The bioimpedance signal represents dependence of resistivity on extracellular fluid conductivity (intravascular fluid [blood and lymph], interstitial and transcellular fluid components) and intracellular fluid conductivity in the body when the high frequency current (e.g., 20-100 kHz) is transferred through the tissues of the human body.

The wearable device (610) may also comprise a power supply unit (655). The power supply unit (655) may include a battery or a rechargeable battery for providing power to components of the wearable device (610).

The wearable device (610) may also include a processing unit (605). In various embodiments, the processing unit (605) may be implemented as a hardware utilizing either a combination of microprocessor(s), specially designed application-specific integrated circuits, programmable logic devices, or system on chip configured to run an operation system and various applications. The processing unit (605) may be configured to analyze signals from the electrodes (635a-635f).

In some embodiments, the wearable device (610), particularly the processing unit (605), may comprise a memory module (615) to store sensor data, application data, and instructions to be executable by the processing unit (605).

The wearable device (610) may further comprise a communication unit (645). The communication unit (645) may include a Global System for Mobile communications module, a Wi-Fi^{™} module, a Bluetooth^{™} module, a near field communication module, ANT+, modem module, and the like. The communication unit (645) can be intended to communicate with a data network (640) such as the Internet, a Wide Area Network (WAN), a Local Area Network (LAN), a cellular network, and so forth, to send a data, for example, sensor data and send and receive messages concerning the health condition of the user.

Additionally, the wearable device (610) can comprise, connected to the processing unit (605), a temperature (Temp) unit, photoplethysmogram (PPG) unit, electrodermal (galvanic or sympathetic skin) activity (EDA) unit, accelerometer (ACC) unit, barometer (BARO) unit, gyroscope (GYRO) unit and other signal sensing or detecting units.

The environment (600) may further comprise a computing device (630). The computing device (630) may include a personal computer (PC), a laptop, a smartphone, a tablet PC, a personal wearable device, and so forth. The computing device (630) can be configured to receive, via the communication unit (645) and/or data network (640), sensor data from the bioimpedance (BIA) unit (625), temperature (Temp) unit, photoplethysmogram (PPG) unit, electrodermal (galvanic or sympathetic skin) activity (EDA) unit, accelerometer (ACC) unit, barometer (BARO) unit, gyroscope (GYRO) unit and other signal sensing or detecting units of the wearable device (610) and process the sensor data to improve the estimation of a discrepancy between a current physiological state of the user and the homeostatic state of the user, its origin and compensation status, and an at least one effective way of treatment of a homeostatic state decompensation, correction (recovery or restoration) of a homeostatic state deviation, protection of a homeostatic state, and/or promotion of homeostatic state resiliency against current and/or future challenges and events.

In some embodiments, the signals from the bioimpedance unit (625) and other Temp, PPG, EDA ACC, BARO, and GYRO units can be first pre-processed by the processing unit (605) and then sent to the computing device (630) for further analysis. The pre-processing of the signals may include transformation of the signal from analog to digital formats, filtering the signals to reduce or suppress noise and artifacts in the signals, to extract carrier of the signals and different bands modulating the signals including amplitude, magnitude, frequency and phase demodulations, resampling and smoothing of the signals, and mathematical processing of the signals including averaging them and/or their derivatives over respective periods of interest.

The environment (600) may further include a server or computing cloud (620). The server or computing cloud (620) can comprise computing resources (hardware and software) available at a remote location and accessible over the data network (640). The server or computing cloud (620) can be communicatively coupled to the wearable device (610) via the data network (640). The server or computing cloud (620) can be shared by multiple user(s). In certain embodiments, the server or computing cloud (620) may include one or more server farms/clusters including a collection of computer servers that can be co-located with network switches and/or routers.

The server or computing cloud (620) may be configured to receive, via a web platform (in some embodiments manually delivered), the communication unit (645) and data network (640), sensor data form the bioimpedance unit (625) and other physiological, behavioral, and environmental sensor units of the wearable device (610) and process the sensor data to estimate a discrepancy between a current physiological state of the user and the homeostatic state of the user, its origin and compensation status, and an at least one effective way of treatment of a homeostatic state decompensation, correction (recovery or restoration) of a homeostatic state deviation, protection of a homeostatic state, and/or promotion of homeostatic state resiliency against current and/or future challenges and events.

The signals from the bioimpedance unit (625) and other physiological sensor units can be first pre-processed by the processing unit (605) and then sent to the server or computing cloud (620) for further analysis. The pre-processing of the signals may include transformation of the signal from an analog to digital format, filtering the signals to reduce or suppress noise and artifacts in the signals, to extract carrier of the signals and different bands modulating the signals including amplitude, magnitude, frequency and phase demodulations, resampling and smoothing of the signals, and mathematical processing of the signals including averaging them and/or their derivatives over respective periods of interest.

The server or computing cloud (620) may be further configured to provide results of analysis of the sensor data and warning and recommendation messages to the user of the wearable device (610) and other authorized users. The authorized users may monitor the result of analysis using one or more applications of computing devices associated with the authorized users. The authorized users may be associated with health care institutions, medical insurance companies, sport organizations, and so forth.

Figures 14-17 show locations of electrodes (635a-635f) for measuring bioimpedance of tissues of a body. Specifically, Figure 14 shows locations (704, 705) of internal electrodes (635a-635d) for local bioimpedance measurement (740) in short circuits (707) and positions of arms and external electrodes (635e-635f) for segmental bioimpedance measurement (711) in long circuits (706) closed by the user's hand contacted to external electrodes (635e-635f) or by a wire (708) connecting pairs of internal electrodes of the device located on two opposite limbs (arms or forearms) in different embodiments.

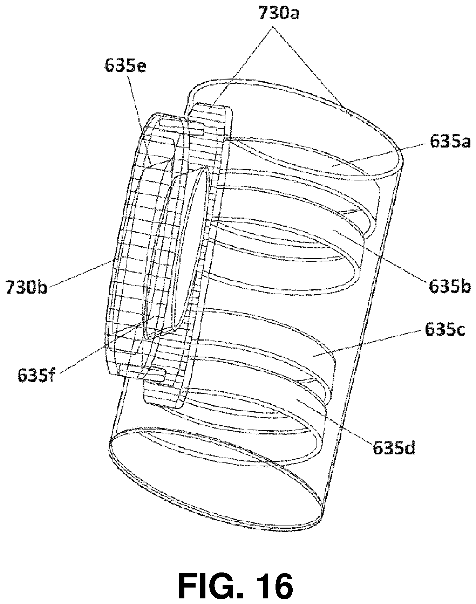

Figure 16 shows variants of external flexible electrodes (635e, 635f) for contact only a part of a limb (e.g., a palm surface) or for its full contact by surrounding a limb (e.g., around a hand). The part of the device with external electrodes for a long bioimpedance circuit (706) may be integrated as a permanent part in one unit along with the part of the device with internal electrodes for a short bioimpedance circuit (707) or be a separate part or a component with an easy attachment and detachment to the part with internal electrodes with their hardware and software re- and de-linking for communication to simplify exploitation of the device with its wearing, cleaning, replacing, and fixing.

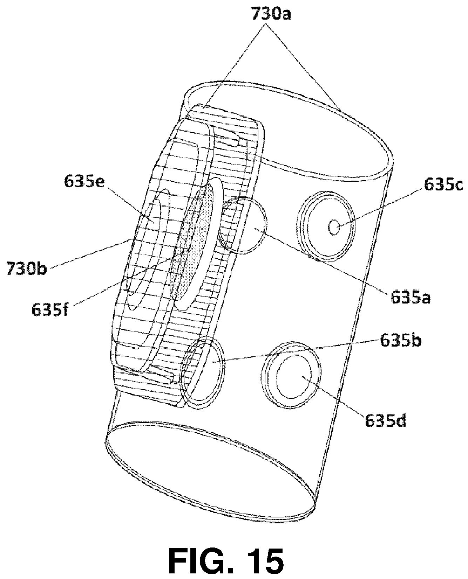

Figures 15-17 show electrodes (635a-d) for local (a short circuit (707)) bioimpedance measurement, and electrodes (635e-f) for segmental between-arm (a long circuit (706)) bioimpedance measurement of tissues of a body when using a wearable device (610) for monitoring a homeostatic state of the present disclosure, according to an example embodiment. In the device (610) of the present disclosure, the electrodes may be attached to a shoulder, to a brachium (705), to a forearm (704), or a wrist of the user. Figure 18 shows a cross-section (810) of the brachium (705) along a surface (840) for one of example location of internal and external metal plate electrodes (Figures 19, 20, 21) and for internal and external flexible electrodes (Figures 9A-9C).

Figure 19 shows a path (825) of one example embodiment and Figure 22 shows a superficial path (924) and a deeper path (925) of another example embodiment of electrodes with the current flowing (electrical lines) between the current electrodes (635a and 635d), which create a voltage difference measured by the voltage electrodes (635b and 635c).

Figure 19 shows pairs of electrodes (635a-d) located on opposite sides of the brachium (705). Specifically, the current electrodes (635a and 635d), which create current, are located on opposite sides of the brachium (705) at a distance from each other sufficient for a current path through local skeletal muscle tissues and small and medium arteries and arterioles. Voltage electrodes (635b and 635c), which measure a voltage difference created by the current electrodes (635a and 635d), are located on the opposite sides of the brachium (705) at a distance from each other sufficient for measuring a voltage difference created by the current pathed through local skeletal muscle tissues and small and medium arteries and arterioles. Such placement of the electrodes (635a-d) ensures the passage of the current through all tissues of the brachium (705), namely, skin (850), adipose tissue (860), muscle tissue (870), and/or bone tissue (880). Also, the current passes through small and medium blood vessels (890) that are present inside the brachium (705).

Figure 22 shows a superficial path (924) and a deeper path (925) of another example embodiment of flexible electrodes around a limb with the current flowing between the current electrodes (635a and 635d), as measured by the voltage electrodes (635b and 635c). Such placement of the electrodes (635a-d) ensures the passage of the current through all tissues of the brachium (705), namely, skin (850), adipose tissue (860), muscle tissue (870), and/or bone tissue (880). Also, the current passes through small and medium blood vessels (890) that are present inside the brachium (705).

During bioimpedance measurement performed with the electrodes (635a-d) placed as shown in Figure 19 or Figure 22, the collected bioimpedance data (represented by measures of impedance, resistance, reactance, and phase angle effects of the circuits) include the information relating to local water volume in the portion of the body around the limb with the common (for local and central hemodynamic) body water osmolality of the user homeostatically maintained. Based on the collected bioimpedance data, a conclusion about a condition of the peripheral blood pressure regulation mechanism by total peripheral vascular activity and a deviation of the peripheral hydration from the homeostatic state of the body can be made. The deviation of the peripheral hydration from the homeostatic state may be considered to be one of indicators of the health of the user and a part of the mechanism leading to decompensation of the homeostatic state and a risk for hypo-volemic essential hypertension.

Figures 20 and 23 show pairs of metal plate or flexible electrodes (635e-f) located externally and isolated from the brachium (705) and internal electrodes (635a-d). Specifically, the current electrode (635e) creates long circuit alternating current from the second limb (arm) touching it by hand (fingers, palm, or wrist) or forearm through the trunk or the thorax of the body to the internal current electrodes (635a-b) located on the brachium or forearm of the first limb being isolated from contacts with the external electrodes and the second limb.

An external voltage electrode (635f) located close to the external current electrode (635e) and internal voltage electrodes (635b and/or 635c) measure a voltage difference created by the current electrodes (635e and 635a-b). Such placement of the current external electrode (635e) and internal electrodes (635a-b) ensures the passage of the current through all tissues of the body from one arm to another arm or hand through the thorax including large blood vessels such as aorta and superior vena cava and the heart with atriums and ventricles for the central bioimpedance measurement.

During bioimpedance measurement performed with the electrodes (635a-f) placed as shown in Figures 19-20 or Figures 22-23, the collected bioimpedance data include the information relating to central or total water volume in the segment from arm-to-arm or arm-to-hand of the body with the common (for local and central hemodynamic) body water osmolality of the user homeostatically maintained. Based on the collected bioimpedance data, a conclusion about a condition of the central blood pressure regulation mechanism by cardiac- and thoracic-pumping activities and a deviation of the central hydration from the homeostatic state of the body can be made. The deviation of the central hydration from the homeostatic state may be considered to be one of indicators of the health of the user and a part of the mechanism leading to decompensation of the homeostatic state and a risk for hyper-volemic essential hypertension.

Figure 25 shows a system (1000) for estimating and/or monitoring the deviation of the body hydration from a homeostatic state, for obtaining and delivering information of its origin and compensation status, and an at least one effective way of treatment of a homeostatic state decompensation, correction (recovery or restoration) of a homeostatic state deviation, protection of a homeostatic state, and/or promotion of homeostatic state resiliency against current and/or future challenges and events, according to an example embodiment. The whole system can be located in one place as the wearable device (i.e., with all processes from data acquiring through data processing, analysis, and storage to alert and message in situ) or parts of the system can be distributed between various locations from the wearable device to local network (with internet-of-things devices) and internet-cloud servers with various distribution of different functions (i.e., data acquiring, processing, programming, storage, computing, and modeling), or the system can work in both the single local, and distributed modes or in modes with functions variously mixed between local and distributed places on-line and off-line depending on situations.

The system (1000) may comprise a model(s) module (1030), physiological state estimation module (1040), and an alert module (1050). The physiological state estimation module (1040) may collect data associated with a user to calculate, for example, an individual homeostatic range using MESOR and 2A as a reference of homeostatic state deviations. Specifically, the physiological state estimation module (1040) may collect bioimpedance data (1010), PPG, Temp, EDA, ACC, BARO, GYRO, and anthropometric data (1020).

The bioimpedance data (1010) can be measured by the wearable device (610) as shown in Figure 13 or in some embodiments may be obtained from readings of raw bioimpedance data presented by the wearable or a stationary device (with the bioimpedance measurement procedure described for the wearable device) using different options including various displays for transferring data to the system in situ or postponed using a web platform (e.g., entered manually by the user, a nurse, or a doctor), or via a wired or wireless communication using photo-camera or cameras of smartphone or laptop with optical character recognition option for digits, letters, and words reading from the displayed data. The anthropometric data (1020) may be entered by the user or assessed automatically (such as distance between electrodes and limb's circumference asl well as between-arm distance as measures related to user's weight and height using features of flexible or adjustable materials, data from bioimpedance, ACC, BARO or other sensors, and/or additional electrical, electromagnetic, optical, and/or acoustic signals generated and measured by the wearable device (610)).

The bioimpedance data (1010) and the anthropometric data (1020) may be provided as an input to the model(s) module (1030) to obtain various parameters associated with the user, such as changes in body water or fluid volume, concentration of electrolytes in the body fluids, a level of the deviation of peripheral and central hydration status from an individual homeostatic range assessed by MESOR and 2A (based on which hypohydration, dehydration, or hyperhydration can be determined), information of its origin and compensation status, and an at least one effective way of treatment of a homeostatic state decompensation, correction (recovery or restoration) of a homeostatic state deviation, protection of a homeostatic state, and/or promotion of homeostatic state resiliency against current and/or future challenges and events (e.g., an amount of a dehydrate or rehydrate and its electrolyte concentration needed to restore the level of hydration and osmolality, a water-salt balance of a human body, a content of glucose and proteins in body fluids, salt restriction diet, thermal or cooling bathing, exercise type and intensity, antihypertensive medication [e.g., diuretics and vasodilators] recommendations, and so forth) according to an example embodiment.

Specifically, the parameters associated with the user may be calculated based on the collected bioimpedance data (1010), data from other sensors such as PPG, Temp, EDA, ACC, BARO, GYRO, and anthropometric data (1020). For example, the water volume or body hydration distribution regulated separately at periphery (by the vascular mechanism) and centrally (by the cardio-respiration mechanism) but in according to the total body hypo-, eu-, or hyper-hydration state which, in turn, regulated separately by (i) perspiration with water elimination (using a skin moistening or water accumulation mechanism in epidermis) in contrast to water/electrolyte conservation mechanism in skin dermis and (ii) diuresis in contrast to water/electrolyte retention mechanism in the kidneys, is one of the main indicators showing whether the state of the human body is healthy.

In view of this, the peripheral and central body water distribution can be used to determine a degree of discrepancy of the current state of the human body from a homeostatic state of human body (water volume in general and its peripheral and central distribution as in an example embodiment), its origin (total water hyper- or hypovolemic state as in an example embodiment), its compensation or decompensation state (coupling or decoupling of peripheral and central distribution of water volume as in an example embodiment) and a way of bouncing back to the homeostatic state (recommendations for water conservation or elimination medications and/or other interventions as in an example embodiment).

While the total water volume as a homeostatic hydration status can be determined by specific bioimpedance measures assessing water and electrolyte conservation in skin attributed to a dermis condition related to sweat production and cutaneous blood flow activity and assessed by a dermis reactance component of a total body impedance), a body hydration distribution at periphery and centrally with its compensated or decompensated balance status can better be assessed by a body resistance or a body complex component of bioimpedance as presented in an example embodiment.

Thus, total body water deficits and proficits with respect to individual homeostatic level are regulated by body water conservation and body water volume peripheral-to-central redistribution mechanisms and can separately be assessed by a skin derma bioimpedance (reactance) measure and by central and peripheral (local) bioimpedance (resistance) measures differently correlated with amplitudes of body hydration and blood pressure circadian variations and nighttime body hydration and blood pressure dipping, orthostatic hypotension and orthostatic tachycardia, orthostatic intolerance with dizziness, nausea and fainting, as well as body dehydration/rehydration with increase/decrease of thirst and dry mouth sensations.

Additionally, the bioimpedance data (1010) associated with other short-term processes such as respiration, pulse volume, heart rate, and muscle metabolic activity variabilities are taken into consideration by the model(s) module (1030). Therefore, upon measuring the bioimpedance data (1010) associated with the other processes in tissues of the body affected water volume assessment locally and the other processes in the whole body affected water volume assessment in general, a more advanced parameter that shows a level of deviation of hydration from a homeostatic state of the whole body of the user can be determined.

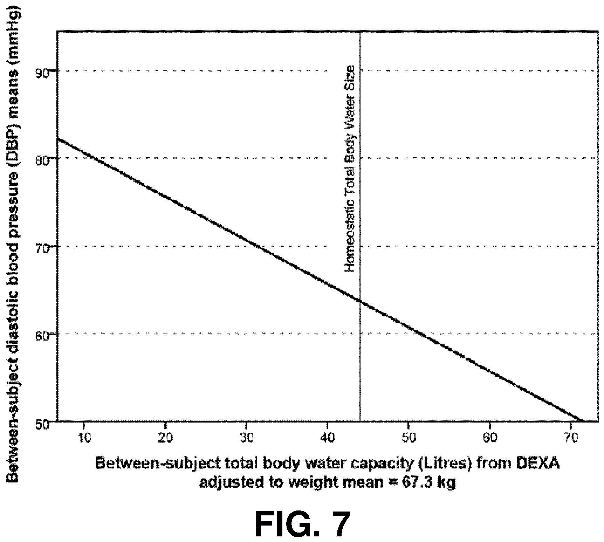

In an example embodiment, a statistical model may be used to determine normal levels (i.e., values corresponding to a healthy state of a human body) of values of body parameters for specific anthropometric data of people such as height and weight (Figures 6 and 7). The statistical model can be further used in the bioimpedance analysis of the user. If a normal water volume is known for a person, an amount of a rehydrate or dehydrate a person needs to consume or to eliminate to reach homeostatic state may be determined.

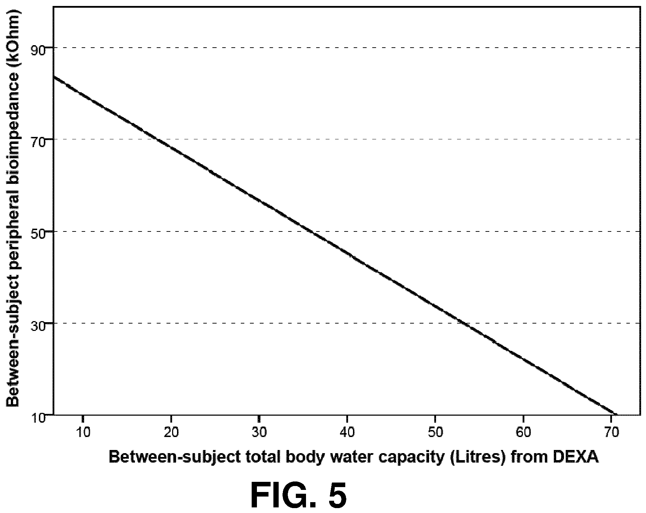

In the statistical model, a population of people (e.g., 100 people) can be selected and anthropometric data (e.g., a height, a weight, an age, a sex, race/ethnicity) of the people can be collected. The people can be studied using a reference method, e.g., via a computer tomography scanning such as dual-energy x-ray absorptiometry (DEXA), to determine an amount of water relative to the total mass of the body. Based on the bioimpedance data, the anthropometric data, and the amount of water data, a regression analysis can be used to calculate a dependency between a combination of bioimpedance data (an independent variable), the anthropometric data (an independent or moderation variable), and the water volume of the human body (a dependable variable) (Figures 4 and 5).

The dependency can be used to obtain one or more regression models that map the combination of bioimpedance data and the anthropometric data into physiological parameters of the body, such as a peripheral and central water balance, a level of hydration, total water of the body, and so forth. Then, the regression models can be used to determine effects of compensated or not compensated peripheral and central water balance regulation on individual systolic and diastolic blood pressure levels (Figures 1-3 and 8) and the risk for hyper- or hypovolemic essential hypertension with the most effective way of its reduction and homeostatic state protection and promotion for sustainable healthy life of the user.

Then, the regression models can be used by the wearable device (610) shown in Figure 13 to estimate physiological parameters of a person using the wearable device (610). The wearable device (610) may store anthropometric data of the person. The wearable device (610) can measure bioimpedance data of the person. The anthropometric data and the measured bioimpedance data can be fed to the regression models to obtain current values for physiological parameters of the person's body.

Compared with the traditional approach of regression models with population normal range as a health-related reference, a more advanced method of the assessment of individual homeostatic normal range as a health-related reference may be implemented in the present disclosure. In this case, the model(s) module (1030) may analyze the bioimpedance data (1010), data from other sensors and anthropometric data (1020) with consideration of the fact that individual homeostatic normal parameters of the body depend on many factors producing their variation within individual normal ranges for respective periods of time or specific situations and the challenge can be resolved by calculating such indicators as MESOR and 2A as in an example embodiment.

In the present disclosure, the model(s) module (1030) analyzes and physiological state estimation module (1040) collects a variability of physiological parameters depending on (with reference or markers to) various seasonal, daytime (circadian), menstrual, feeding/fasting, drinking, working/non-working, vacation, and other cycles, clocks, events, or/and body activities, random and non-random environment conditions, social, psychological (emotional and cognitive), behavioral and physical events, to which the body must respond in a converse manner if in normal body hydration condition with compensated deviations from the homeostatic setpoint within its range (detected by a positive correlation coefficient or correspondence in balancing of peripheral and central water volume distribution as in an example embodiment) as compared to how the body reacts in hypo-hydration and over-hydration conditions with uncompensated deviations from the homeostatic setpoint outside its range (detected by a negative or zero correlation coefficient of peripheral and central water volume distribution as indicators of poor correspondence or disbalance as in an example embodiment). Based on this analysis, the bioimpedance data are calibrated and an individual homeostatic range of fluctuations of body parameters or a permanent deviation of the data from a homeostatic state with volumetric origin of essential hypertension and respective recommendations are determined.

Additionally, each person has his own homeostatic range of hydration level, which may range from 39% to 84% of a body mass depending on the anthropometric data, such as a sex, a menstrual cycle phase in women, an age, a race, and external factors such as activity, occupation, current weather, local climate, and so forth. This defines the water reserve that an individual may acutely or chronically lose to preserve his homeostatic hydration level without affecting the health (i.e., keep his blood pressure level within a normal range as in an example embodiment) during circadian fluctuations in body water.

The bioimpedance may be sensitive to water or fluid distribution between different parts of the body. For example, a peripheral blood flow may reduce by 40% by high vasoconstriction as a compensation mechanism, but a central blood flow may reduce by 23% by a decreased cardiac output, when a body mass may decrease by 5% due to water loss or elimination. Therefore, considerable changes in a body water distribution obtained by the bioimpedance method in some body's parts may correspond to relatively small changes in a body mass as a proxy of body water loss or gain. This may explain normal circadian fluctuations of body water between about 48% and about 80% in healthy subjects, as assessed by the bioimpedance measures obtained from some peripheral parts of the body.

Additionally, alterations in responses of a heart rate, vascular activity, stroke volume, renal, and sweat gland activity to various challenges such as postural change, environmentally affected local body (e.g., skin on an arm) and whole-body (e.g., warm or cold clothes, warming by sauna or cooling by air conditioner) temperature changes, and water/food intake may affect momentary bioimpedance measurements and thus water balance assessments that should be taken into account to improve reliability of homeostatic state indicators.

It was found in studies carried out by the present inventors that effects of compensated and not compensated homeostatic (dis)balance in water volume distribution between peripheral and central parts of the body (hyper-, hypo- or iso-hydration or water (dis)balance in body tissues) on blood pressure level may be assessed by bioimpedance measures such as body resistance at 50kHz frequency of electrical current with a path in short and/or long circuit as in example embodiment. The findings also showed that resistance to this single frequency alternating electrical current with a path in short and long circuit can separately predict diastolic and systolic blood pressure levels using a local (e.g., on a part of an arm) and segmental (e.g., arm-to-arm) bioimpedance models, respectively (Figures 1, 2, 8, and 3). These findings also showed that changes in bioimpedance measurements and thus the water volumes in these parts influences blood pressure in different directions (Figures 1, 8 and 3).

Another study of the inventors showed consistency between a total body water volume measured by the DEXA and a bioimpedance resistance component as an inverse proxy measure of hydration state in peripheral and central parts of the body (Figures 4 and 5). The same study also confirmed that water volume balance of the body as assessed by DEXA had an opposite effect on blood pressure regulation peripherally (i.e., on diastolic blood pressure as a pressure mainly regulated by vascular activity) and centrally (i.e., on systolic blood pressure as a pressure mainly regulated by cardiac activity) (Figures 6 and 7). The homeostatic total body water volume for humans with height = 1.67 m and weight = 67.3 kg was found to be about 44 litres.

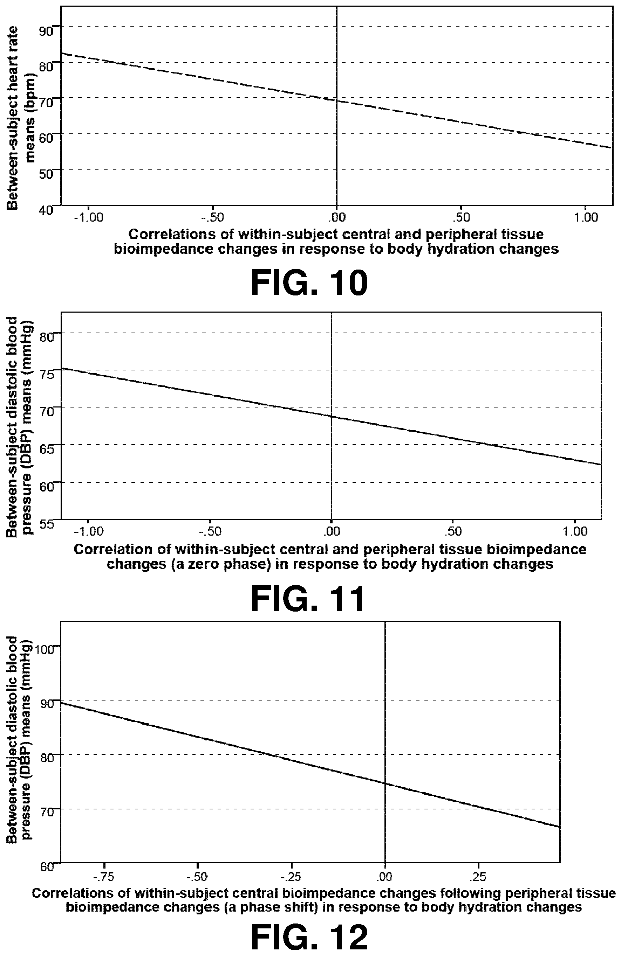

Another study of one of the inventors shows that high correspondence in water balance distribution in the body as assessed by a high positive correlation of peripheral and central body water volumes using peripheral and central bioimpedance measures in response to hydration status daily challenges such as dehydration and rehydration indicates that any deviations from hydration status as a homeostatic body state is compensated in this case and does not lead to health problems as essential hypertension development (Figures 10 and 11). In contrast, inconsistency in balance between peripheral and central water volumes as assessed by the bioimpedance measures (negative or zero correlations and cross-correlations) can lead to increased blood pressure and thus to a risk of essential hypertension development (Figures 10-12).

Thus, the studies show that coupled and uncoupled balance in body hydration status between peripheral and central parts of the body as a compensated and decompensated status of homeostatic state of body water regulation can be monitored by the combined local and segmental bioimpedance measurements if the bioimpedance is calibrated based on populational between-subject variability and/or on within-subject variability (with calculation of such variables as MESOR and 2A) of individual water balance responses to hydration status challenges such as various daily, weekly, monthly, and/or seasonal body dehydration and rehydration events.

Thus, in an example embodiment, two reference models may be used in the assessment of the hydration balance level as an indicator of two hypo- and hyper-volemic mechanisms of blood pressure regulation.

The first model is an experimentally obtained bioimpedance model associated with a body water balance distributed peripherally and centrally for predicting a main mechanism of blood pressure regulation and origin of its deviation from normal or healthy condition with a populational reference obtained by DEXA or various radioisotope dilution techniques capable to assess body water volumes with adjustment based on a body weight and a body length in groups with specific sex, race/ethnicity and so on.

The second, more advanced model with an additional compensation status assessment is a bioimpedance model experimentally or ecologically determined in particular individuals as users or in specific homogeneous populations with calculation of such variables as MESOR and 2A and a measure of concordance, correspondence, coherence, or cross-correlation in variabilities of data of body water balance distributed peripherally and centrally in response to minutely, hourly, daily, weekly, monthly, seasonal and other regular (e.g., cycling) and irregular (random and non-random) events and accumulated during periods of time for predicting a main mechanism of blood pressure regulation, origin of its deviation from normal or healthy level or range.

Thus, the second model may have also a reference to within-subject MESOR, 2A, and correspondence (coherence, cross-correlation and so on) measures of the individual bioimpedance data of the water balance with other physiological (e.g., , cardiac [blood pressure and heart rate], respiration and so on), behavioral, psychological, social, and environmental data accumulated (collected) during the same periods of time or during periods of the time shifted with forward and backward lags (Figures 9 and 12) for further improving of individual homeostatic level and range calculation (e.g., MESOR and 2A variables) and compensated and decompensated deviations from them.

The physiological state estimation module (1040) may use the model(s) module (1030) to compare current physiological parameters of the user to normal physiological parameters determined based on the statistical models. Based on the comparison, the physiological state estimation module (1040) may estimate a discrepancy between the current physiological state of the user and the homeostatic state of the user.

In an example embodiment, the normal level and range can be adjusted using a statistical model based on an aggregated data of hydration status (i.e., as an average, median or so on) monitored per a defined period from minutes to months and years.

Figure 26 is a flow chart of example method (1100) for monitoring a homeostatic state, according to some example embodiments. The method (1100) may be performed by wearable device (610) of Figure 13. The device (610) can be integrated in the environment (600) of Figure 13. The wearable device (610) can be worn by a user. The method may include periodically or irregularly sensing, by a bioimpedance unit (625) and via internal electrodes (635a-f) configured to touch a limb, a bioimpedance of the user at operation (1105), and via internal and external electrodes (635a-f) configured to touch two limbs of the user, a bioimpedance of the user at operation (1110). In an example embodiment, the bioimpedance unit (625) and the processing unit (605) may be integrated into a layer of a material configured to be secured around the limb of the user.

The method may continue at operation (1115) with determining at least one physiological parameter of the user by the processing unit (605) communicatively coupled to the bioimpedance unit (625). The at least one physiological parameter of the user may be determined based on the bioimpedance or on the bioimpedance and anthropometric data of the user. The anthropometric data may include one or more of the following: a height of the user, a weight of the user, an age of the user, a sex of the user, and so forth. The at least one physiological parameter may include an individual water volume (level of hydration), water balance, or water balance deviation from a homeostatic level or range in peripheral tissues within a portion of the body around the limb of the user and/or in tissues and spaces of central organs, as well as their compensated or non-compensated correspondence.

In some example embodiments, the at least one physiological parameter may be determined using a regression model. The regression model may be generated based on statistical data sets of values of the bioimpedance, values of the anthropological data, and values of the at least one physiological parameter. The values of the at least one physiological parameter may be obtained via a populational reference method, such as computer tomography scanning, or individual reference method after accumulating or collecting bioimpedance data along with other physiological, behavioral, psychological, clinical (medical), social, and environmental data.

The method may further include estimating, by the processing unit, a discrepancy between a current physiological state of the user and the homeostatic state of the user, its origin and compensation status, and an at least one effective way of treatment of a homeostatic state decompensation, correction (recovery or restoration) of a homeostatic state deviation, protection of a homeostatic state, and/or promotion of homeostatic state resiliency at operation (1120). The estimation may be performed based on the at least one physiological parameter.

The method may continue with issuing, by the processing unit (605) and based on the discrepancy, at least one alert or message with recommendations concerning the current physiological state of the user at operation (1125). In an example embodiment, the at least one alert or message may be provided in a form of a notification on a computing device.

The method (1100) may further include determining an amount of a dehydrate or rehydrate, a method of intervention with a treatment or correction including more effective treatment of hypertension recommendations as a clinical outcome of the hydration status condition by water conservation or elimination (vasodilators or diuretics, salt non-restricted or restricted diet), or hydration status protection with its resiliency against current and/or future events by the processing unit based on the level of hydration and its deviation from a homeostatic level. Based on the information, the processing unit (605) may issue a recommendation to the user or another responsible person (a relative, doctor, or nurse) or a (healthcare) service in methods for coping with hydration challenge, for recovering after it, for protecting against it, or for promoting resilience mechanisms.

Figure 27 illustrates an exemplary computing system (1200) that may be used to implement embodiments described herein. The computing system (1200) can be implemented in the contexts of the likes of network (640), computing device (630), and server or computing cloud 620. The exemplary computing system (1200) of Figure 27 may include one or more processors (1210) and memory (1220). Memory (1220) may store, in part, instructions and data for execution by the one or more processors (1210). Memory (1220) can store the executable code when the exemplary computing system (1200) is in operation. The exemplary computing system (1200) of Figure 27 may further include a mass storage (1230), portable storage (1240), one or more output devices (1250), one or more input devices (1260), a network interface (1270), and one or more peripheral devices (1280).

The components shown in Figure 27 are depicted as being connected via a single bus (1290). The components may be connected through one or more data transport means. The one or more processors (1210) and memory (1220) may be connected via a local microprocessor bus, and the mass storage (1230), one or more peripheral devices (1280), portable storage (1240), and network interface (1270) may be connected via one or more input/output buses.

Mass storage (1230), which may be implemented with a solid state disk, a magnetic disk, or an optical disk drive, is a non-volatile storage device for storing data and instructions for use by a solid state disk, a magnetic disk, or an optical disk drive, which in turn may be used by one or more processors (1210). Mass storage (1230) can store the system software for implementing embodiments described herein for purposes of loading that software into memory (1220).

Portable storage (1240) may operate in conjunction with a portable non-volatile storage medium, such as a solid state disk (SSD), compact disk (CD) or digital video disc (DVD), to input and output data and code to and from the computing system (1200) of Figure 27. The system software for implementing embodiments described herein may be stored on such a portable medium and input to the computing system (1200) via the portable storage (1240).

One or more input devices (1260) provide a portion of a user interface. The one or more input devices (1260) may include an alphanumeric keypad, such as a keyboard, for inputting alphanumeric and other information, or a pointing device, such as a mouse, a trackball, a stylus, or cursor direction keys. Additionally, the computing system (1200) as shown in Figure 27 includes one or more output devices (1250). Suitable one or more output devices (1250) include speakers, printers, network interfaces, and monitors.

Network interface (1270) can be utilized to communicate with external devices, external computing devices, servers, and networked systems via one or more communications networks such as one or more wired, wireless, or optical networks including, for example, the Internet, intranet, LAN, WAN, cellular phone networks (e.g., Global System for Mobile communications network, packet switching communications network, circuit switching communications network), Bluetooth radio, and an IEEE 802.11-based radio frequency network, among others. Network interface (1270) may be a network interface card, such as an Ethernet card, optical transceiver, radio frequency transceiver, sound transceiver, or any other type of device that can send and receive information. Other examples of such network interfaces may include Bluetooth^{®}, 3G, 4G, and WiFi^{®} radios in mobile computing devices as well as a Universal Serial Bus (USB).

One or more peripheral devices (1280) may include any type of computer support device to add additional functionality to the computing system. The one or more peripheral devices (1280) may include a modem or a router.

The components contained in the exemplary computing system (1200) of Figure 27 are those typically found in computing systems that may be suitable for use with embodiments described herein and are intended to represent a broad category of such computer components that are well known in the art. Thus, the exemplary computing system (1200) of Figure 27 can be a personal computer, handheld computing device, telephone, mobile computing device, workstation, server, minicomputer, mainframe computer, or any other computing device. The computer can also include different bus configurations, networked platforms, multi-processor platforms, and so forth. Various operating systems (OS) can be used including UNIX, Linux, Windows, Macintosh OS, Palm OS, and other suitable operating systems.

Some of the above-described functions may be composed of instructions that are stored on storage media (e.g., computer-readable medium). The instructions may be retrieved and executed by the processor. Some examples of storage media are memory devices, tapes, disks, and the like. The instructions are operational when executed by the processor to direct the processor to operate in accord with the example embodiments. Those skilled in the art are familiar with instructions, processor(s), and storage media.

It is noteworthy that any hardware platform suitable for performing the processing described herein is suitable for use with the example embodiments. The terms "computer-readable storage medium" and "computer-readable storage media" as used herein refer to any medium or media that participate in providing instructions to a central processing unit (CPU) for execution. Such media can take many forms, including, but not limited to, non-volatile media, volatile media, and transmission media. Non-volatile media include, for example, a solid state, optical, or magnetic disks, such as a fixed disk. Volatile media include dynamic memory, such as RAM. Transmission media include coaxial cables, copper wire, and fiber optics, among others, including the wires that include one embodiment of a bus. Transmission media can also take the form of acoustic or light waves, such as those generated during radio frequency and infrared data communications. Common forms of computer-readable media include, for example, a floppy disk, a flexible disk, a hard disk, magnetic tape, any other magnetic medium, a CD-read-only memory (ROM) disk, DVD, any other optical medium, any other physical medium with patterns of marks or holes, a RAM, a PROM, an EPROM, an EEPROM, a FLASHEPROM, any other memory chip or cartridge, a carrier wave, or any other medium from which a computer can read.

Various forms of computer-readable media may be involved in carrying one or more sequences of one or more instructions to a CPU for execution. A bus carries the data to system RAM, from which a CPU retrieves and executes the instructions. The instructions received by system RAM can optionally be stored on a fixed disk either before or after execution by a CPU.

## Claims

1. A wearable device (610) for monitoring a homeostatic state of a user, that comprises:
- a bioimpedance unit (625), configured to sense at least one tissue of a user, via a first set of electrodes (635a-f) intended to touch a portion of the body around a limb locally or other parts of the body with regions with well-vascularized tissues, and a bioimpedance of whole-body or segmental (thorax or trunk) of the user via a second set of electrodes (635a-f), intended to touch tissues around the respective central parts of the body of the user,
- a processing unit, communicatively coupled to the bioimpedance unit (625), that comprises:
- a memory module (615), that stores anthropometric data of the user,
- a physiological parameter calculation module (1040), connected to the memory module (615), configured to calculate at least one physiological parameter of the user based on the bioimpedance obtained with the sets of electrodes (635a-f) or on the bioimpedance and anthropometric data of the user stored in the memory module (615),
- a discrepancy calculation module configured to estimate, based on the at least one physiological parameter, a discrepancy between a current physiological state of the user and the usual homeostatic state of the user, and
- an alert module (1050), connected to the discrepancy calculation module and configured to, based on the discrepancy existence, issue at least one message with recommendations concerning the current physiological state of the user.

2. The wearable device (610) of claim 1, wherein the bioimpedance unit (625) comprises six electrodes (635a-635f), wherein a first pair and a second pair of electrodes (635a-635f) are intended to touch a portion of the body around a limb locally or other parts of the body with regions with well-vascularized tissues, and a third pair of electrodes (635a-635f) that, in combination with the first or second pair of electrodes (635a-635f) are intended to touch tissues around the respective central parts of the body of the user.

3. The wearable device (610) of claim 1, wherein the bioimpedance unit (625) comprises four electrodes (635a-635f), wherein a first pair and a second pair of electrodes (635a-635f) are intended to touch a portion of the body around a limb locally or other parts of the body with regions with well-vascularized tissues.

4. The wearable device (610) of claim 1, wherein the bioimpedance unit (625) comprises four electrodes (635a-635f), wherein a first pair and a second pair of electrodes (635a-635f) are intended to touch tissues around the respective central parts of the body of the user.

5. The wearable device (610) of claim 1, wherein the anthropometric data of the user is selected between: user's body weight, height, age, sex, distance between the electrodes, circumference of the portion of the body part and sectional area.

6. The wearable device (610) of claim 1, wherein the physiological parameters are selected between: absolute or relative volume of fluids (water) in tissues within a portion of the body part of the user, a level of hydration with balance of water, electrolytes in tissues within a portion of the body part of the user and a level of compensated or decompensated correspondence of water or hydration balance between the tissues within a portion of the body part of the user.

7. The wearable device (610) of claim 1, wherein it additionally comprises a support layer holding the bioimpedance unit (625) and the processing unit (605).

8. The wearable device (610) of claim 1, wherein the processing unit (605) additionally comprises a communication unit (645), intended connect to an external computing device (630).

9. The wearable device (610) of claim 1, wherein it additionally comprises a sensor connected to the processing unit (605) and selected between: a temperature (Temp) unit, photoplethysmogram (PPG) unit, electrodermal (galvanic or sympathetic skin) activity (EDA) unit, accelerometer (ACC) unit, barometer (BARO) unit and gyroscope (GYRO) unit.

10. A method for monitoring bias from a homeostatic state of a user, that comprises the steps of:
- periodically or irregularly sensing a local and central bioimpedance of the user, using the device of any of the previous claims,
- determining at least one physiological parameter of the user based on the bioimpedance or on the bioimpedance and anthropometric data of the user,
- estimating, based on the at least one physiological parameter, a discrepancy between a current physiological state of the user and the usual homeostatic state of the user, for the same condition or for a general or specific population reference,
- determining the origin of the discrepancy,
- determining, based on the absolute or relative levels of total, regional, and local hydration, an amount of body water to dehydrate or to rehydrate for removing the discrepancy in the homeostatic state,
- issuing at least one message with recommendations concerning the current physiological state of the user.

11. The method of claim 10, wherein the local and central bioimpedance measurements to obtain the at least one physiological parameter are conducted both in a same measurement trial or each separately in their own measurement trials.

12. The method of claim 10, wherein the values of the at least one physiological parameter are obtained using a reference method selected between computer tomography scanning and a dilution of a radioisotope.

13. The method of claim 10, wherein the at least one physiological parameter is determined using a mathematical model obtained from a range of between-subjects measures in population or from a range of within-subject measures in the individual.

14. The method of claim 13, wherein the mathematical model is generated based on data sets of values of the bioimpedance, values of the anthropological data, and values of the at least one physiological parameter.

15. The method of claim 10, wherein the message includes at least a recommendation to the user selected between: taking the actions to rehydrate, taking a substance to loss the respective amount of solute or water volume, taking a vasodilator and taking a diuretic.
